(19) Europäisches Patentamt
European Patent Office
Office européen des brevets

(11) Publication number: **0 127 177**
**B1**

(12) # EUROPEAN PATENT SPECIFICATION

(45) Date of publication of patent specification: **21.12.88**

(51) Int. Cl.$^4$: **C 07 F 9/65,** A 61 K 31/675

(21) Application number: **84106131.0**

(22) Date of filing: **29.05.84**

(54) **(3-Acylamino-2-oxo-1-azetidinyl)oxyrmethylrphosphinic acids.**

(30) Priority: **31.05.83 US 449643**

(43) Date of publication of application:
**05.12.84 Bulletin 84/49**

(45) Publication of the grant of the patent:
**21.12.88 Bulletin 88/51**

(84) Designated Contracting States:
**AT BE CH DE FR GB IT LI LU NL SE**

(56) References cited:
**EP-A-0 061 765**
**GB-A-2 125 794**

(73) Proprietor: **E.R. Squibb & Sons, Inc.**
**Lawrenceville-Princeton Road**
**Princeton, N.J. 08540 (US)**

(72) Inventor: **Koster, William H.**
**Box 409 R.D. 1 Welisewitz Rd.**
**Ringoes New Jersey (US)**
Inventor: **Breuer, Hermann**
**Sauerzapfstrasse 5**
**D-8411 Schönhofen (DE)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 127 177 B1

## Description

2-oxo-1-(((substituted sulfonyl)amino)carbonyl)azetidines having a phosphinic or phosphonic substituent in the 1-position and an acrylamino substituent in the 3-position and a process for their preparation are disclosed in EP—A—61 765. These compounds exhibit atnibacterial activity.

β-Lactams having an

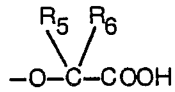

substituent (and esters and salts thereof) in the 1-position and an acrylamino substituent in the 3-position including the analogues having an optionally protected $NH_2$ group in the 3-position are known from GB—A—2 125 794.

Antibacterial activity is exhibited by β-lactams having the formula

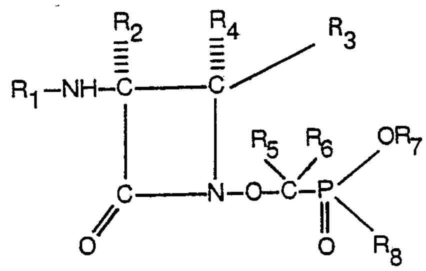

(I)

and pharmaceutically acceptable salts thereof. As used in formula I, and throughout the specification, the symbols are as defined below.

$R_1$ is acyl;

$R_2$ is hydrogen or methoxy;

$R_3$ and $R_4$ are the same or different and each is hydrogen, alkyl, alkenyl, alkynyl, cycloalkyl, phenyl, substituted phenyl or a 4, 5, 6 or 7-membered heterocycle (referred to hereinafter as $R_9$) or one of $R_3$ and $R_4$ is hydrogen and the other is azido, halomethyl, dihalomethyl, trihalomethyl, alkoxycarbonyl, alkenyl, alkynyl, 2-phenylethenyl, 2-phenylethynyl, carboxyl, $—CH_2X_1$ [wherein $X_1$ is azido, amino ($—NH_2$), hydroxy, alkanoylamino, phenylcarbonylamino, (substituted phenyl)carbonylamino, alkylsulfonyloxy, phenylsulfonyloxy, (substituted phenyl)sulfonyloxy, phenyl, substituted phenyl, cyano,

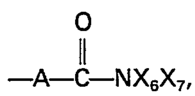,

$—S—X_2$, or $—O—X_2$ (wherein A, $X_2$, $X_6$ and $X_7$ are as hereinafter defined)], $—S—X_2$ or $—O—X_2$ [wherein $X_2$ is alkyl, substituted alkyl, phenyl, substituted phenyl, phenylalkyl, (substituted phenyl)alkyl, alkanoyl, phenylalkanoyl, (substituted phenyl)alkanoyl, phenylcarbonyl, (substituted phenyl)carbonyl, or heteroarylcarbonyl]).

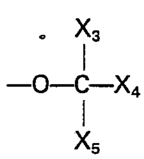 or 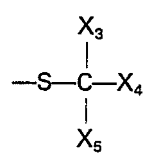

[wherein one of $X_3$ and $X_4$ is hydrogen and the other is hydrogen or alkyl, or $X_3$ and $X_4$ when taken together with the carbon atom to which they are attached form a cycloalkyl group; and $X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl

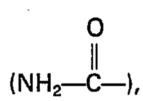,

(substituted amino) carbonyl, or cyano ($—C{\equiv}N$)], or

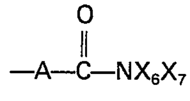

(wherein A is —CH=CH—, $—(CH_2)_n—$, $—CH_2—O—$, $—CH_2—NH—$, or $—CH_2—S—CH_2$, n is 0, 1 or 2, and $X_6$ and $X_7$ are the same or different and each is hydrogen, alkyl, phenyl or substituted phenyl, or $X_6$ is hydrogen and $X_7$ is amino, substituted amino, acylamino or alkoxy, or $X_6$ and $X_7$ when taken together with the nitrogen atom to which they are attached form a 4, 5, 6 or 7-membered heterocycle);

$R_5$ and $R_6$ are the same or different and each is hydrogen, alkyl, alkenyl, alkynyl, phenyl, substituted phenyl, cycloalkyl or $R_9$, or $R_5$ and $R_6$ together with the carbon atom to which they are attached are cycloalkyl or $R_9$, or one of $R_5$ and $R_6$ is hydrogen and the other is azido, halomethyl, dihalomethyl, trihalomethyl, alkoxycarbonyl, alkenyl, alkynyl, 2-phenylethenyl, 2-phenylethynyl, carboxyl, $—CH_2X_1$, $—S—X_2$, $—O—X_2$, or

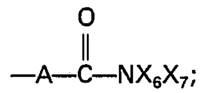;

$R_7$ is hydrogen, alkyl, substituted alkyl, phenyl, substituted phenyl, 1-(ethoxycarbonyloxy) ethyl, 1,3-dihydro-3-oxo-1-isobenzofuranyl,

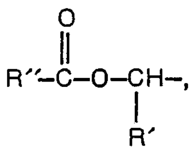, or 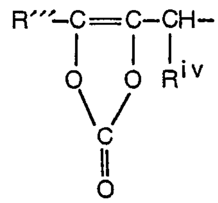

wherein R′ is hydrogen or alkyl, R″ is alkyl or phenyl, R‴ is hydrogen, methyl or phenyl and $R^{iv}$ is hydrogen or together R‴ is $—(CH_2)_3—$ or $—(CH_2)_5—$; and

$R_8$ is hydroxy, alkoxy, (substituted alkyl)oxy, phenyloxy, (substituted phenyl)oxy, alkyl, substituted alkyl, phenyl, substituted phenyl, heteroaryl, amino, substituted amino, alkylthio, (substituted alkyl)thio, phenylthio, (substituted phenyl)thio, or

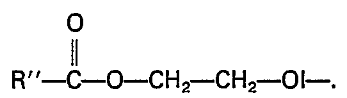.

Listed below are definitions of various terms used to describe the β-lactams of this invention. These definitions apply to the terms as they are used throughout the specification (unless they are otherwise limited in specific instances) either individually or as part of a larger group.

The terms "alkyl" and "alkoxy" refer to both straight and branched chain groups. Those groups having 1 to 10 carbon atoms are preferred.

The terms "cycloalkyl" and "cycloalkenyl" refer to cycloalkyl and cycloalkenyl groups having 3, 4, 5, 6 or 7 carbon atoms.

The term "substituted alkyl" refers to alkyl groups substituted with one, or more, azido, amino ($—NH_2$), halogen, hydroxy, carboxy, cyano, alkoxycarbonyl, aminocarbonyl, alkanoyloxy, alkoxy, phenyloxy, (substituted phenyl)oxy, $R_9$-oxy, mercapto, alkylthio, phenylthio, (substituted phenyl)thio, alkylsufinyl, or alkylsulfonyl groups.

The terms "alkanoyl", "alkenyl", and "alkynyl" refer to both straight and branched chain groups. Those groups having 2 to 10 carbon atoms are preferred.

The terms "halogen" and "halo" refer to fluorine, chlorine, bromine and iodine.

The term "substituted phenyl" refers to a phenyl group substituted with 1, 2 or 3 amino($—NH_2$), halogen, hydroxyl, trifluoromethyl, alkyl (of 1 to 4 carbon atoms), alkoxy (of 1 to 4 carbon atoms), carbamyl, or carboxyl groups.

The expression "a 4, 5, 6 or 7-membered heterocycle" (referred to as "$R_9$") refers to substituted and unsubstituted, aromatic and nonaromatic groups containing one or more nitrogen, oxygen of sulfur atoms. Exemplary substituents are oxo(=O), halogen, hydroxy, nitro, amino, cyano, trifluoromethyl, alkyl of 1 to 4 carbons, alkoxy of 1 to 4 carbons, alkylsulfonyl, phenyl, substituted phenyl, 2-furylimino

( 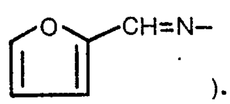 ).

benzylimino and substituted alkyl groups (wherein the alkyl group has 1 to 4 carbons). One type of "4, 5, 6 or 7-membered heterocycle" is the "heteroaryl" group. The term "heteroaryl" refers to those 4, 5, 6 or 7-membered heterocycles which are aromatic.

Exemplary heteroaryl groups are substituted and unsubstituted pyridinyl, furanyl, pyrrolyl, thienyl, 1,2,3-triazolyl, 1,2,4-triazolyl, imidazolyl, thiazolyl, thiadiazolyl, pyrimidinyl, oxazolyl, triazinyl, and tetrazolyl. Exemplary nonaromatic heterocycles (i.e., fully or partially saturated heterocyclic groups) are substituted and unsubstituted azetinyl, oxetanyl, thietanyl, piperidinyl, piperazinyl, imidazolidinyl, oxazolidinyl, pyrrolidinyl, tetrahydropyrimidinyl, dihydrothiazolyl and hexahydroazepinyl. Exemplary of the substituted 4, 5, 6 or 7-membered heterocycles are 1-alkyl-3-azetinyl, 2-oxo-1-imidazolidinyl, 3-alkylsulfonyl-2-oxo-1-imidazolidinyl, 3-benzylimino-2-oxo-1-imidazolidinyl, 3-alkyl-2-oxo-1-imidazolidinyl, 3-phenyl (or substituted phenyl)-2-oxo-1-imidazolidinyl, 3-benzyl-2-oxo-1-imidazolidinyl, 3-(2-aminoethyl)-2-oxo-1-imidazolidinyl, 3-amino-2-oxo-1-imidazolidinyl, 3-[(alkoxycarbonyl)amino]-2-oxo-1-imidazolidinyl, 3-[2-[(alkoxycarbonyl)amino]ethyl]-2-oxo-1-imidazolidinyl, 2-oxo-1-pyrrolidinyl, 2-oxo-3-oxazolidinyl, 4-hydroxy-6-methyl-2-pyrimidinyl, 2-oxo-1-hexahydroazepinyl, 2-oxo-3-pyrrolidinyl, 2-oxo-3-furanyl, 2,3-dioxo-1-piperazinyl, 2,5-dioxo-1-piperazinyl, 4-alkyl-2,3-dioxo-1-piperazinyl, and 4-phenyl-2,3-dioxo-1-piperazinyl.

The term "substituted amino" refers to a group having the formula $—NY_1Y_2$ wherein $Y_1$ is hydrogen, alkyl, phenyl, substituted phenyl, phenylalkyl or (substituted phenyl)alkyl, and $Y_2$ is alkyl, phenyl, substituted phenyl, phenylalkyl, (substituted phenyl)alkyl, hydroxy, cyano, alkoxy, phenylalkoxy, or amino ($—NH_2$).

The term "acyl" refers to all organic radicals derived from an organic acid (i.e., a carboxylic acid) by removal of the hydroxyl group. Certain acyl groups are, of course, preferred but this preference should not be viewed as a limitation of the scope of this invention. Exemplary acyl groups are those acyl groups which have been used in the past to acylate β-lactam antibiotics including 6-aminopenicillanic acid and derivatives and 7-aminocephalosporanic acid and derivatives; see, for example, *Cephalosporins and Penicillins,* edited by Flynn, Academic Press (1972), German Offenlegungsschrift 2,716,677, published October 10, 1978, Belgian patent 867,994, published December 11, 1978, United States patent 4,152,432, issued May 1, 1979, United States patent 3,971,778, issued July 27, 1976, United States patent 4,172,199, issued October 23, 1979, and British patent 1,348,894, published March 27, 1974. The portions of these references describing various acyl groups are incorporated herein by reference. The following list of acyl groups is presented to further exemplify the term "acyl"; it should not be regarded as limiting that term. Exemplary acyl groups are:

(a) Aliphatic groups having the formula

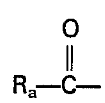

wherein $R_a$ is alkyl; cycloalkyl; alkoxy; alkenyl; cycloalkenyl; cyclohexadienyl; or alkyl or alkenyl substituted with one or more halogen, cyano, nitro, amino, mercapto, alkylthio, or cyanomethylthio groups.

(b) Carbocyclic aromatic groups having the formula

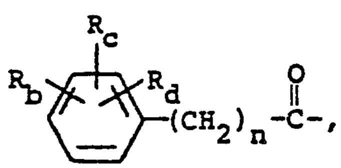 , 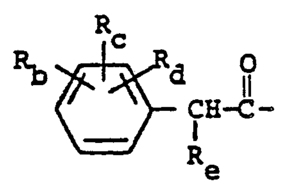 , 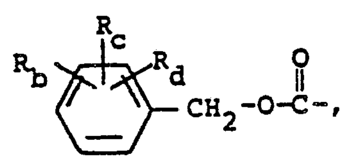 ,

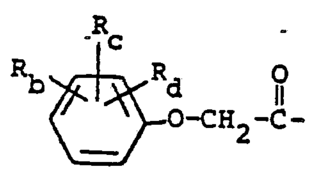 , 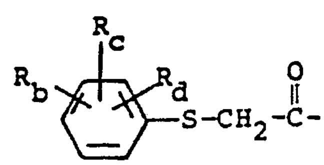 or 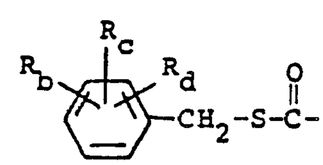

wherein n is 0, 1, 2 or 3; $R_b$, $R_c$, and $R_d$ each is independently hydrogen, halogen, hydroxyl, nitro, amino, cyano, trifluoromethyl, alkyl of 1 to 4 carbon atoms, alkoxy of 1 to 4 carbon atoms or aminomethyl; and $R_e$ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thioxomethyl]thio.

Preferred carbocyclic aromatic acyl groups include those having the formula

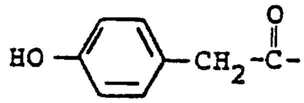 , 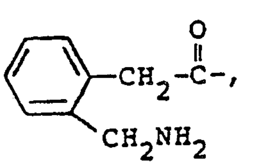 , 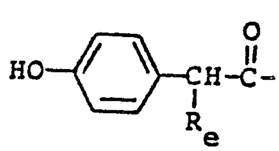

($R_e$ is preferably a carboxyl salt or sulfo salt) and

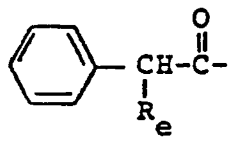

($R_e$ is preferably a carboxyl salt or sulfo salt).

c) Heteroaromatic groups having the formula

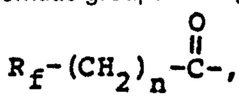 , 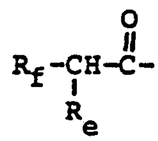 , 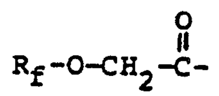 ,

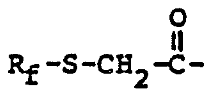 , 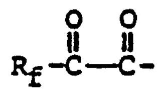 ,

wherein n is 0, 1, 2 or 3; $R_e$ is as defined above; and $R_f$ is a substituted or unsubstituted 5-, 6- or 7-membered heterocyclic ring containing 1, 2, 3 or 4 (preferably 1 or 2) nitrogen, oxygen and sulfur atoms. Exemplary heterocyclic rings are thienyl, furyl, pyrrolyl, pyridinyl, pyrazolyl, pyrazinyl, thiazolyl, pyrimidinyl, thiadiazolyl and tetrazolyl. Exemplary substituents are halogen, hydroxyl, nitro, amino, protected amino, cyano, trifluoromethyl, alkyl of 1 to 4 carbon atoms, alkoxy of 1 to 4 carbon atoms, or

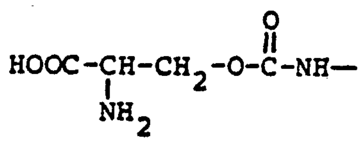 .

Preferred heteroaromatic acyl groups include those groups of the above formulas wherein $R_f$ is 2-amino-4-thiazolyl, 2-amino-5-halo-4-thiazolyl, 4-aminopyrimidin-2-yl, 5-amino-1,2,4-thiadiazol-3-yl, 2-thienyl, 2-furanyl, or 6-aminopyridin-2-yl.

(d) [[(4-Substituted-2,3-dioxo-1-piperazinyl)carbonyl]amino]arylacetyl groups having the formula

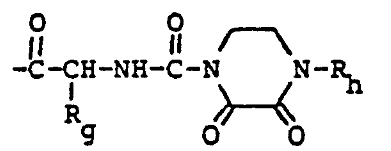

wherein $R_g$ is an aromatic group (including carbocyclic aromatics such as those of the formula

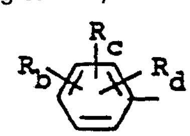

and heteroaromatics as included within the definition of $R_f$); and $R_h$ is alkyl, substituted alkyl (wherein the alkyl group is substituted with one or more halogen, cyano, nitro, amino or mercapto groups), arylmethyleneamino (i.e., —N=CH—$R_9$ wherein $R_g$ is as defined above), arylcarbonylamino (i.e.,

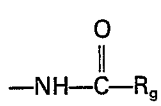

wherein $R_g$ is as defined above) or alkylcarbonylamino.

Preferred [[(4-substituted-2,3-dioxo-1-piperazinyl)carbonyl]amino]arylacetyl groups include those wherein $R_h$ is ethyl, phenylmethyleneamino or 2-furylmethyleneamino.

(e) (Substituted oxyimino)arylacetyl groups having the formula

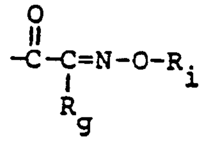

where $R_g$ is as defined above and $R_i$ is hydrogen alkyl, cycloalkyl, alkylaminocarbonyl, arylaminocarbonyl (i.e,

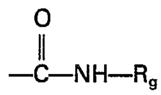

wherein $R_g$ is as defined above) or substituted alkyl (wherein the alkyl group is substituted with one or more halogen, cyano, nitro, amino, mercapto, alkylthio, aromatic group (as defined by $R_g$), carboxyl (including salts thereof), amido, alkoxycarbonyl, phenylmethoxycarbonyl, diphenylmethoxycarbonyl, hydroxyalkoxyphosphinyl, dihydrotyphosphinyl, hydroxy(phenylmethoxy)phosphinyl, or dialkoxyphosphinyl substituents).

Preferred (substituted oxyimino)arylacetyl groups include those wherein $R_g$ is 2-amino-4-thiazolyl. Also preferred are those groups wherein $R_i$ is methyl, ethyl, carboxymethyl, 1-carboxy-1-methylethyl, 2,2,2-trifluoroethyl or 1-carboxycyclopropyl.

(f) (Acylamino)arylacetyl groups having the formula

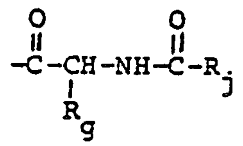

wherein $R_g$ is as defined above and $R_j$ is

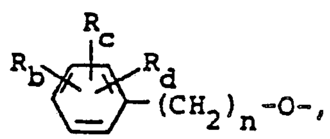

amino, alkylamino, (cyanoalkyl)amino, amido, alkylamido, (cyanoalkyl)amido,

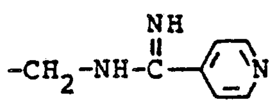

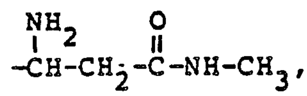

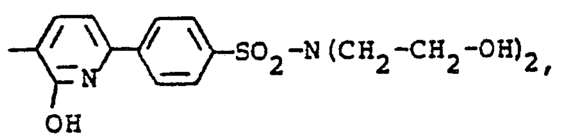

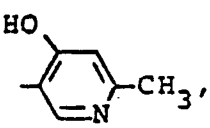

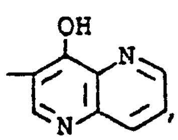

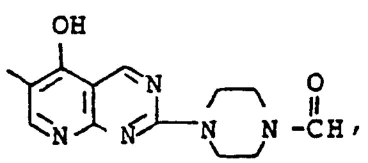 or

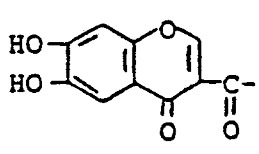 .

Preferred (acylamino)arylacetyl groups of the above formula include those groups wherein $R_j$ is amino or amido. Also preferred are those groups wherein $R_g$ is phenyl or 2-thienyl.

(g) [[[3-Substituted-2-oxo-1-imidazolidinyl]carbonyl]amino]arylacetyl groups having the formula

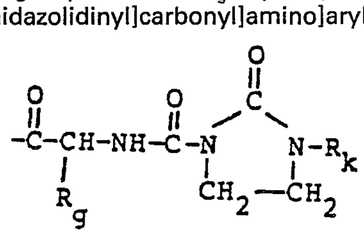

wherein $R_g$ is as defined above and $R_k$ is hydrogen, alkylsulfonyl, arylmethyleneamino (i.e., $—N=CH—R_g$ wherein $R_g$ is a defined above),

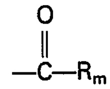

(wherein $R_m$ is hydrogen, alkyl or halogen substituted alkyl), aromatic group (as defined by $R_g$ above), alkyl or substituted alkyl (wherein the alkyl group is substituted with one or more halogen, cyano, nitro, amino or mercapto groups).

Preferred [[3-substituted-2-oxo-1-imidazolidinyl]carbonyl]amino]arylacetyl groups of the above formula include those wherein $R_g$ is phenyl or 2-thienyl. Also preferred are those groups wherein $R_k$ is hydrogen, methylsulfonyl, phenylmethyleneamino or 2-furylmethyleneamino.

The compounds of this invention form basic salts with various inorganic and organic bases which are also within the scope of this invention. Such salts include ammonium salts, alkali metal salts, alkaline earth metal salts, salts with organic bases, e.g. dicyclohexylamine, benzathine, N-methyl-D-glucamine, hydrabamine and the like. The pharmaceutically acceptable salts are preferred, although other salts are also useful, e.g. in isolating or purifying the product.

Some of the compounds of this invention may be crystallized or recrystallized from solvents containing water. In these cases water of hydration may be formed. This invention contemplates stoichiometric hydrates as well as compounds containing variable amounts of water that may be produced by processes such as lyophilization.

β-Lactams having an

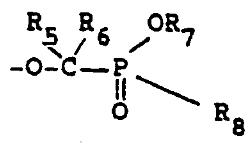

substituent in the 1-position and an amino or acylamino substituent in the 3-position contain at least one chiral center — the carbon atom (in the 3-position of the β-lactam nucleus) to which the amino or acylamino substituent is attached. This invention is directed to those β-lactams which have been described above, wherein the stereochemistry at the chiral center in the 3-position of the β-lactam nucleus is the same as the configuration at the carbon atom in the 6-position of naturally occurring penicillins (e.g., penicillin G) and as the configuration at the carbon atom in the 7-position of naturally occurring cephamycins (e.g., cephamycin C).

Also included within the scope of this invention are racemic mixtures which contain the above-described β-lactams.

β-Lactams having an

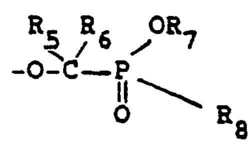

substituent in the 1-position of the β-lactam nucleus and an acylamino substituent in the 3-position of the β-lactam nucleus have activity against a range of gram-negative and gram-positive organisms.

The compounds of this invention can be used as agents to combat bacterial infections (including urinary tract infections and respiratory infections) in mammaliam species, such as domesticated animals (e.g., dogs, cats, cows, horses, and the like) and humans.

For combating bacterial infections in mammals, a compound of this invention can be administered to a mammal in need thereof in an amount of about 1.4 mg/kg/day to about 350 mg/kg/day, preferably about 14 mg/kg/day to about 100 mg/kg/day. All modes of administration which have been used in the past to deliver penicillins and cephalosporins to the site of the infection are also contemplated for use with the novel family of β-lactams of this invention. Such methods of administration include oral intravenous, intramuscular, and as a suppository.

The β-lactams of this invention can be prepared from am amino acid having the formula

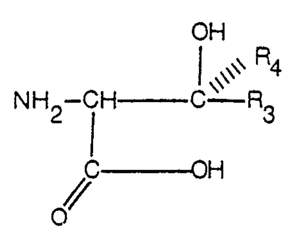

II

The amino group is first protected with a classical protecting group (e.g., *t*-butoxycarbonyl, benzyloxycarbonyl, *o*-nitrophenylsulfenyl, etc), yielding a compound having the formula

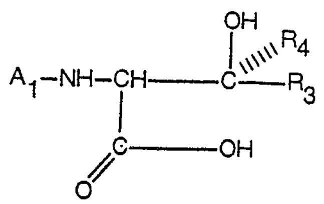

III

In formula III, and throughout the specification, the symbol "$A_1$" refers to a nitrogen protecting group.

The carboxyl group of a protected amino acid of formula III is then reacted with an amine salt having the formula IV

$$Y—O—NH_3^{\oplus}Cl^{\ominus}$$

In formula IV, and throughout the specification, the symbol "Y" refers to benzyl, pivaloyl, $—CH_2CH(NHA_2)CO_2$alkyl ($A_2$ is an amino protecting group), *t*-butyl, *p*-nitrobenzyl, benzhydryl, 2-cyanoethyl, 2-trimethylsilylethyl, trichloroethyl, *p*-anisyl, *inter alia*. The reaction proceeds in the presence of a coupling agent such as 1-ethyl-3-(3-dimethylaminopropyl)carbodiimide or dicyclohexylcarbodiimide, and yields a compound having the formula

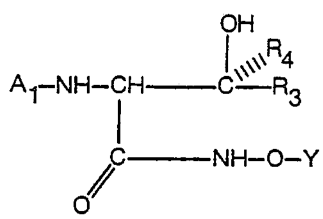

V

The hydroxyl group of a compound of formula V is converted to a leaving group, using, for example, a classical reagent such as methanesulfonyl chloride (methansulfonyl is referred to hereinafter as "Ms").

The fully protected compound having the formula

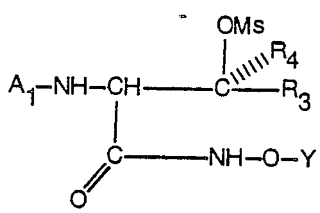

VI

is cyclized by treatment with base, e.g., potassium carbonate. The reaction is preferably carried out in an organic solvent such as acetone, under reflux conditions, and yields a compound having the formula

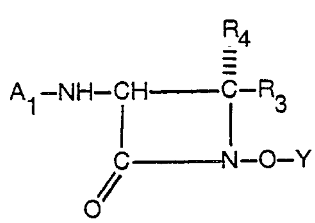

VII

Alternatively, cyclization of a compound of formula V can be accomplished without first converting the hydroxyl group to a leaving group. Treatment of a compound of formula V with triphenylphosphine and diethylazodicarboxylate or carbon tetrachloride, yields a compound of formula VII.

Both of the methods disclosed above for ring closure of a compound of formula V result in the inversion of the stereochemistry of the $R_3$ and $R_4$ substituents.

Selective reduction of a compound of formula VII (using catalytic hydrogenation if Y is benzyl or by treatment with a base such as sodium sulfide or sodium hydroxide if Y is pivaloyl, or with DBU if Y is

$-CH_2CH(NHA_2)CO_2$alkyl yields the corresponding compound having the formula

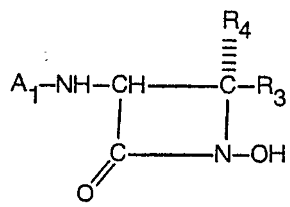

VIII

Compounds of formula VIII are described in GB—A—2 125 794 and some are also described in *J.A.C.S., 102,*:7026 (1980).

Alkylation of a hydroxamic acid of formula VIII with an activated form of a compound having the formula

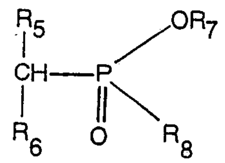

IX

can be accomplished by first generating the anion of the hydroxamic acid with a suitable base, and then reacting the resulting compound with an activated form of an acetic acid derivative of formula IX. Activated and protected forms of compounds of formula IX have the formula

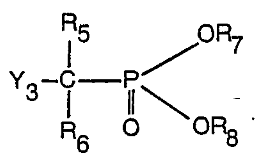

IXa

wherein $Y_3$ is a suitable leaving group, such as a triflate group, or other reactive leaving group well known in the art. The above alkylation procedure has been described as a two step sequence, but both steps can be performed simultaneously. The resulting product has the formula

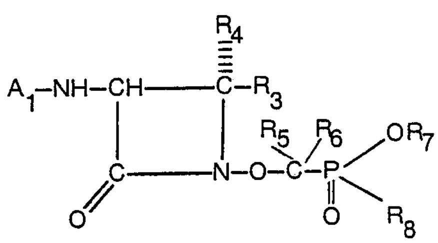

X

Deprotection of the 3-amino substituent of a compound of formula X can be accomplished using art-recognized techniques. If, for example, the protecting group is *t*-butoxycarbonyl, trifluoroacetic acid can be used to deprotect the amino group. If the protecting group is benzyloxycarbonyl, catalytic (e.g., palladium on charcoal) hydrogenation can be used. If the protecting group is *o*-nitrophenylsulfenyl, *p*-toluenesulfonic acid can be used in combination with *p*-thiocresol. The deprotected compound has the formula

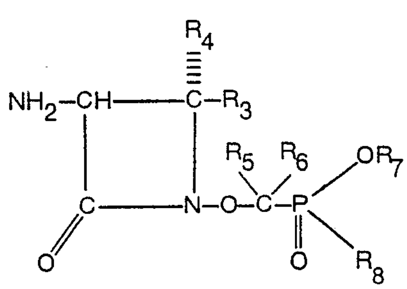

(XI)

and is a key intermediate for preparing the compounds of this invention. The compounds of formula XI form an integral part of this invention.

Well known acylation techniques can be used to convert a compound of formula XI to the corresponding compound having the formula

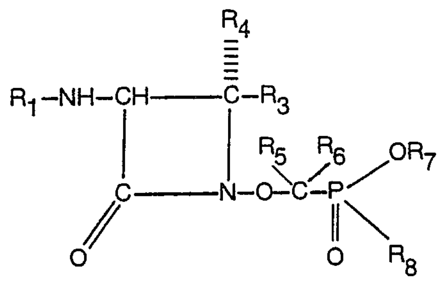

XII

Exemplary techniques include reaction with a carboxylic acid ($R_1$—OH) or corresponding carboxylic acid halide or carboxylic acid anhydride. The reactions with a carboxylic acid proceed most readily in the presence of a carbodiimide such as dicyclohexylcarbodiimide and a substance capable of forming a reactive intermediate *in situ* such as N-hydroxybenzotriazole or 4-dimethylaminopyridine. In those instances wherein the acyl group ($R_1$) contains reactive functionality (such as amino or carboxy groups) it may be necessary to first protect these functional groups, then carry out the acylation reaction, and finally deprotect the resulting product.

Alternatively, the compounds of this invention can be prepared by reacting a compound of formula III with an amine having the formula

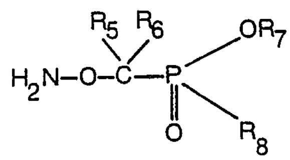

XIII

The reaction proceeds in the presence of a coupling agent such as dicyclohexylcarbodiimide and yields a compound having the formula

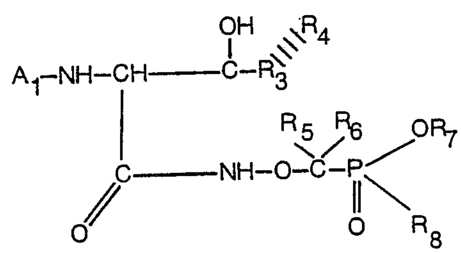

XIV

The amine of formula XIII can be prepared by reacting N-hydroxyphthalimide with an alcohol having the formula

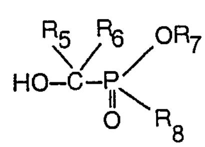

XV

under Mitsunobu conditions (triphenylphosphinediethyldiazodicaboxylate) or with an activated form of the alcohol having the formula

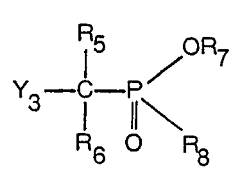

XVa

to yield a compound having the formula

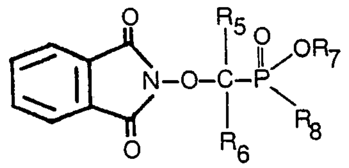

XVI

Cleavage of the phthalimide group in a compound of formula XVI with hydrazine or an alkylhydrazine yields the corresponding amine of formula XIII.

A compound of formula XIV can be cyclized to give a compound of formula X using the procedures described above for the cyclization of a compound of formula VI.

Those compounds of formula XII wherein $R_7$ is alkyl and $R_8$ is alkoxy can be converted to the corresponding diacid having the formula

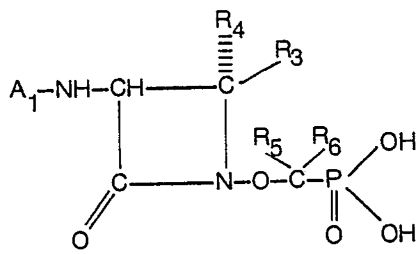

XVII

providing another route for the preparation of the products of formula I wherein $R_7$ is hydrogen and $R_8$ is hydroxyl. Conversion to the diacid can be accomplished using trimethylsilyl bromide in the presence of bis-trimethylsilylacetamide as a drying agent and acid scavenger, and then solvolyzing the resulting bis-silyl ester with water or an alcohol.

Those compounds of formula XII wherein $R_7$ is alkyl and $R_8$ is alkoxy can be heated with thiourea to yield the corresponding monoacid having the formula

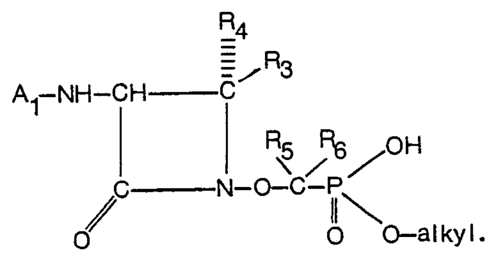

XVIII

providing another route for the preparation of the products of formula I wherein $R_7$ is hydrogen and $R_8$ is alkoxy.

Alternatively, mono-acidic phosphorous derivatives can be obtained from the corresponding monoalkyl esters of formula XII, wherein $R_8$ is alkyl, substituted alkyl, phenyl, substituted phenyl, heteroaryl, amino, or substituted amino, (this subgrouping is referred to hereinafter as $R_{10}$), by treatment with an acid-scavener and drying agent such as bis-trimethylsilylacetamide followed by treatment with trimethylsilyl bromide to yield an intermediate silyl ester having the formula

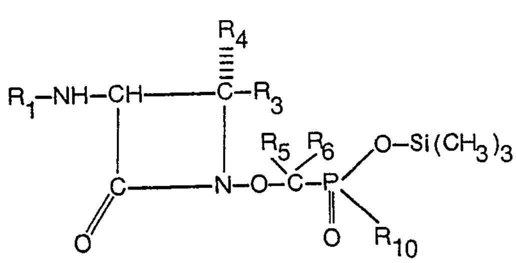

XIX

An intermediate of formula XIX can be treated with an organic or inorganic base in the presence of water or an alcohol to yield a salt of a compound having the formula

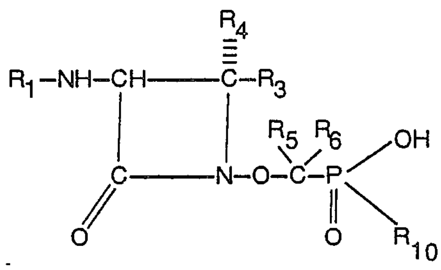

XX

The tetraalkylammonium salt of a compound of formula XX can be treated with an alkyl halide (substituted alkyl)halide, 1-(ethoxycarbonyloxy)ethyl halide, 1,3-dihydro-3-oxo-1-isobenzofouranyl halide.

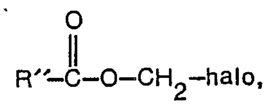 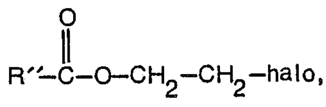 or 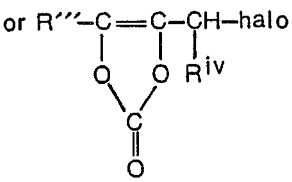

to obtain an ester having the formula

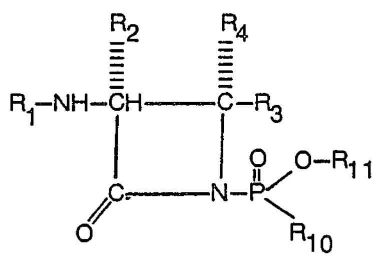

XXI

wherein $R_{11}$ is alkyl, substituted alkyl, 1-(ethoxycarbonyloxy)ethyl, 1,3-dihydro-3-oxo-1-isobenzofuranyl,

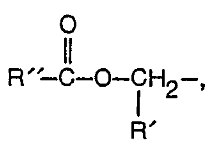 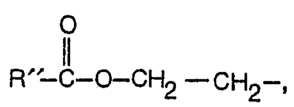 or 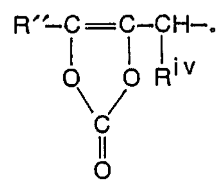

when $R_{10}$ is methoxy or ethoxy i.e., a compound of formula XVIII, this process results in transesterification, yielding the tetraalkylammonium salt of a compound having the formula

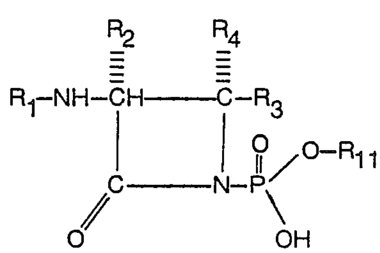

XXII

A compound of formula XXII can be converted to the corresponding compound of formula XXI wherein $R_{10}$ is methoxy or ethoxy by treating the tetraalkylammonium salt of the compound of formula XXII with methyl or ethyl sulfate. Alternatively the tetraalkylammonium salt of the compound of formula XXII can be converted to the corresponding acid on ion-exchange resin and then treated with diazomethane or diazoethane to yield the desired compound of formula XXII wherein $R_{20}$ is methoxy or ethoxy.

The products of formula I wherein $R_2$ is methoxy can be prepared from the corresponding compound of formula VII. Halogenating (preferably chlorinating) the amide nitrogen of a compound of formula VII yields a compound having the formula

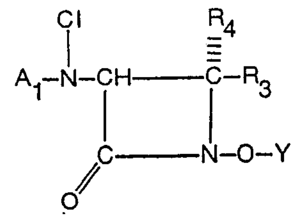

XXIII

Reagents and procedures of N-chlorinating amides are known in the art. Exemplary reagents are *tert.*-butyl hypochlorite, sodium hypochlorite, and chlorine. The reaction can be run in an organic solvent (e.g., a lower alkanoyl such as methanol) or in a two phase solvent system (e.g., water/methylene chloride) in the presence of a base such a sodium borate decahydrate. The reaction is preferably run at a reduced temperature.

Reaction of a compound of formula XXIII with a methoxylating agent, e.g. an alkali metal methoxide, yields a compound (in combination with its enantiomer if $R_3$ and $R_4$ are the same or if XXIII is a racemic mixture) having the formula

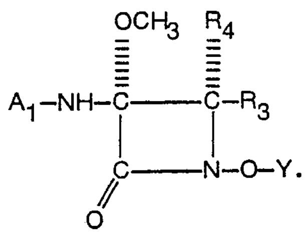

XXIV

The reaction can be run in an organic solvent, e.g., a polar organic solvent such as tetrahydrofuran, at a reduced temperature.

Alternatively, a compound of formula VII can be converted to a compound of formula XXIV using a single step procedure. The methoxylating agent can first be mixed with a compound of formula VII and the N-chlorinating reagent then added to the reaction mixture.

Conversion of a compound of formula XXIV to the desired products of formula I can be accomplished using the procedures described above for the conversion of an intermediate of formula VII to a product of this invention.

The following examples are specific embodiments of this invention.

## Example 1

[3S-[3α(Z),4β]]-[[[3-[[(2-Amino-4-thiazolyl)(methoxyimino)acetyl]amino]-4-methyl-2-oxo-1-azetidinyl]oxy]-methyl]phosphonic acid, potassium salt

A) N-[(Diethoxyphosphinyl)methoxy]-$N^2$-[(1,1-dimethylethoxy)carbonyl]-L-threoninamide

To a solution of N-t-butoxycarbonylamino-L-threonine (3.20 g, 14.6 mmol) in dimethylformamide (35 ml) was added N-hydroxybenzotriazole (1.97 g, 14.6 mmol). The resulting solution was cooled to 0°C, and dicyclohexylcarbodiimide (3.30 g, 16 mmol) was added. The ice bath was removed and the solution was stirred for thirty minutes at room temperature.

After re-cooling the solution to 0°C, diethyl aminooxymethyl phosphonate (2.67 g, 14.6 mmol) in dimethylformamide (25 ml) was added. The solution was then stirred at room temperature for 16 hours. After removal of dimethylformamide at reduced pressure, the crude product (10.6 g) was purified by two successive chromatographies on silica gel (40% acetone/methylene chloride, and 33% acetone/methylene chloride) to afford 1.76 g of the title compound.

B) (3S-*trans*)-[[[3-[[(1,1-Dimethylethoxy)carbonyl]amino]-4-methyl-2-oxo-1-azetidinyl]oxy]methyl]-phosphonic acid, diethyl ester

N-[(Diethoxyphosphinyl)methoxy]-$N^2$-[(1,1-dimethylethoxy)carbonyl]-L-threoninamide (1.38 g, 3.62 mmol) and triphenylphosphine (0.97 g, 3.7 mmol) were placed in a round-bottomed flask, which was driven *in vacuo* for thirty minutes at room temperature. Argon was introduced into the flask, followed by dry tetrahydrofuran (30 ml). The solution was cooled to 0°C and diethylazodicarboxylate (585 µl, 3.7 mmol) was added dropwise. The cooling bath was removed and the solution stirred at room temperature for 16 hours.

Tetrahydrofuran was evaporated at reduced pressure, and the residue triturated with cold ether (30 ml, −10°C) to precipitate triphenylphosphine oxide. Filtration and evaporation at reduced pressure afforded the crude product (1.96 g) which was purified by chromatography on silica gel, eluting with 20% acetone/

methylene chloride. The product obtained in this manner is conveniently separated from the 4-hydroxy-4-methyl-2-pentanone (aldol product) by crystallization from ether:pentane (1:1). The first crop of crystals thus obtained gave 0.61 g of the title compound, melting point 76—80°C. Recrystallization from ether/pentane afforded the product, melting point 85—88°C.

C) [3S-[3α(Z),4β]]-[[[3-[[(2-Amino-4-thiazolyl)(methoxyimino)acetyl]amino]-4-methyl-2-oxo-1-azetidinyl]-oxy]methyl]phosphonic acid, diethyl ester

(3S-*trans*)-[[[3-[[(1,1-Dimethylethoxy)carbonyl]amino]-4-methyl-2-oxo-1-azetidinyl]oxy]methyl]-phosphonic acid, diethyl ester (0.85 g, 2.32 mmol) was weighed into a round-bottomed flask under argon, and cooled to −10°C. A solution of 10% anisole in trifluoroacetic acid at −10°C was added, and the resulting solution was stirred at that temperature for thirty minutes. Evaporation of the volatiles at room temperature with a vacuum pump afforded the crude trifluoroacetic acid salt of the deprotected azetidone starting material which was used directly in the next step.

The activated ester of (Z)-2-amino-α-(methoxyimino)-4-thiazoleacetic acid was prepared by dissolving the acid (0.47 g, 2.32 mmol) and hydroxy benzotriazole (0.31 g, 2.32 mmol) in dimethylformamide (8 ml). This solution was cooled to 0°C and dicyclohexylcarbodiimide (0.48 g, 2.3 mmol) was added. The solution was stirred at room temperature for thirty minutes, and then cooled to 0°C. To this solution of activated ester was added the trifluoroacetic acid salt prepared above, as a solution of dimethylformamide (2.5 ml), followed by two rinses of 2.5 ml and 1.0 ml of dimethylformamide. To this solution was added triethylamine (1.0 ml, 7.2 mmol). The pH was found to be *ca.* 9, and the ice bath was removed.

After stirring for 16 hours at room temperature, dimethylformamide was evaporated *in vacuo.* The residue was treated with 5 ml of acetone, and solid potassium bicarbonate was added to adjust the pH to 6 on moist litmus paper. After filtering the solution to remove inorganic salts and hydroxybenzotriazole salt, the acetone solution was chromatographed on a reverse-phase HP—20 column (25 mm × 15″) using 50% acetone/water to elute 0.47 g of the title compound, melting point 74—78°C.

D) [3S-[3α(Z),4β]]-[[[3-[[(2-Amino-4-thiazolyl)(methoxyimino)acetyl]amino]-4-methyl-2-oxo-1-azetidinyl]-oxy]methyl]phosphonic acid, potassium salt

[3S-[3α(Z),4β]]-[[[3-[[(2-Amino-4-thiazolyl)(methoxyimino)acetyl]amino]-4-methyl-2-oxo-1-azetidinyl]-oxy]methyl]phosphonic acid, diethyl ester (0.10 g, 0.22 mmol) was dissolved in dry methylene chloride (2.0 ml) under argon and cooled to 0°C. To this solution was added bis(trimethylsilyl)acetamide (0.20 m, 1.12 mmol) and the solution was stirred for thirty minutes at 0°C. Trimethylsilylbromide (0.20 ml, 1.5 mmol) was added, and the cooling bath removed. Thin layer chromatography (silica, 50% acetone/methylene chloride) showed no remaining starting material after 2.5 hours, and the volatiles were evaporated *in vacuo,* followed by azeotropic removal of residual trimethylsilyl bromide (two times with 2 ml of toluene) at low pressure.

To the residue thus obtained was added pH 4.0 buffer (10 ml), followed by methylene chloride (10 ml). The mixture was stirred vigorously for fifteen minutes, and the aqueous layer separated, washed with methylene chloride (15 ml) and lyophilized to afford the crude product (0.11 g) which consisted predominantly of one spot by thin layer chromatography ($R_f = 0.05$, 3:1:1 n-butanol:water:acetic acid). Chromatography on HP—20 (25 mm × 12″) using water as the eluting solvent afforded the title compound, melting point 220—225°C, *dec.*

Example 2

[3S-[3α(Z),4β]]-[[[3-[[(2-Amino-4-thiazolyl)(methoxyimino)acetyl]amino]-4-methyl-2-oxo-1-azetidinyl]oxy]-methyl]phosphonic acid, methyl ester, potassium salt

A) (3S-*trans*)-[[[3-[[(1,1-Dimethylethoxy)carbonyl]amino]-4-methyl-2-oxo-1-azetidinyl]oxy]methyl]-phosphonic acid, dimethyl ester

(3S-*trans*)-[[[3-[[(1,1-Dimethylethoxy)carbonyl]amino]-4-methyl-2-oxo-1-azetidinyl]oxy]methyl]-phosphonic acid, diethyl ester (250 mg, 0.68 mmol) was dissolved in dry methylene chloride (5 ml) and cooled to 0°C under an argon atmosphere. Bis-trimethylsilyacetamide (0.5 ml) was added. After stirring for 15 minutes, trimethylsilyl bromide (0.5 ml) was added to the solution, the ice bath removed, and the reaction mixture allowed to warm to room temperature over two hours. The volatiles were removed *in vacuo,* followed by azeotropic removal with toluene (two times with ml). The crude phosphonate bis-trimethylsilyl ester thus prepared was dissolved in dry ether (15 ml), and added to a stirring solution of diazomethane in ether/methanol (ca. 3 mmol, ether/methanol/20 ml/10 ml) at −10°C. After stirring for fifteen minutes, three drops of acetic acid were added, which failed to further discharge the pale yellow color of the reaction mixture. The solvent was then removed at reduced pressure, affording 0.40 g of the crude title compound.

The crude dimethyl phosphonate was purified by chromatography on silica gel (25 mm × 18″), eluting with 4% methanol/ethyl acetate, yielding 170 mg of the title compound of sufficient purity to be used in the next step.

B) (3S-*trans*)-[[[3-[[(1,1-Dimethylethoxy)carbonyl]amino]-4-methyl-2-oxo-1-azetidinyl]oxy]methyl]-phosphonic acid, methyl ester, potassium salt

(3S-*trans*)-[[[3-[[(1,1-Dimethylethoxy)carbonyl]amino]-4-methyl-2-oxo-1-azetidinyl]oxy]methyl]-phosphonic acid, dimethyl ester (170 mg, 0.50 mmol) was dissolved in deuterated acetonitrile (2.5 ml), and thiourea (45 mg, 0.59 mmol) was added. The mixture was heated in a sealed tube overnight at 80°C. Chromatography on Dowex $K^+$ (200—400 mesh, 25 mm × 6''), using water as the eluant, followed by lyophilization of the appropriate fractions afforded the title compound (70 mg).

C) [3S-[3α(Z),4β]]-[[[3-[[(2-Amino-4-thiazolyl)(methoxyimino)acetyl]amino]-4-methyl-2-oxo-1-azetidinyl]-oxy]methyl]phosphonic acid, methyl ester, potassium salt

(3S-*trans*-[[[3-[[(1,1-Dimethylethoxy)carbonyl]amino]-4-methyl-2-oxo-1-azetidinyl]oxy]methyl]-phosphonic acid, methyl ester, potassium salt (70 mg, 0.19 mmol) was dissolved in a solution of 10% anisole in trifluoroacetic acid (2 ml) at −10°C. The solution was stirred for thirty minutes and the volatiles removed at room temperature on a vacuum pump for three hours, to give the crude trifluoroacetic acid salt of the starting azetidinone.

Activated (Z)-2-amino-α-(methoxyimino)-4-thiazoleacetic acid was prepared by dissolving the acid (40 mg, 0.20 mmol) and hydroxybenzotriazole (27 mg, 0.20 mmol) in dimethylformamide (1 ml). After cooling the solution to −10°C, dicyclohexylcarbodiimide (45 mg, 0.22 mmol) was added and the ice bath removed. Stirring was continued for 90 minutes, after which the solution was recooled to −10°C, and the crude trifluoroacetic acid salt prepared above, was added in dimethylformamide (1 ml), followed by two rinses of dimethylformamide (1 ml, 0.5 ml). To this solution was added triethylamine (120 µl, 0.86 mmol). The solution was found to be basic by pH paper, the cooling bath was removed and the solution stirred overnight at room temperature.

Dimethylformamide was removed from the reaction mixture *in vacuo*, and the resulting product treated with water (1.5 ml) to afford a slurry of pH 4. Potassium bicarbonate was added to adjust the pH to 8.5, and the material was chromatographed on Dowex $K^+$ resin (200—400 mesh, 25 mm × 6''). Lyophilization of the fractions containing UV active material afforded 0.70 g of product.

This material was further purified by chromatography on HP—20 resin (25 mm × 8''). Lyophilization of the appropriate fractions afforded 30 mg of the title compound. Analysis of the spectral data for this material indicated contamination of the product by hydroxybenzotriazole and dicyclohexylurea. The product browned at 164°C and decomposed at 210°C.

## Example 3

### [3S-[3α(Z),4β]]-[[[3-[[(2-Amino-4-thiazolyl)(methoxyimino)acetyl]amino]-4-methyl-2-oxo-1-azetidinyl]oxy]-methyl]methylphosphinic acid, potassium salt

A) [Hydroxymethyl]methylphosphinic acid, ethyl ester

Ethyl methylphosphinate (9.41 g, .087 mole) was heated to 85°C under nitrogen. Dried paraformaldehyde (2.16 g, .078 mole) was added, and the reaction mixture was heated at 85—95°C for 5 minutes and then at 78°C for 2 hours. The product was distilled at 128°C/0.9 mm to yield 7.92 g of the title compound.

B) [[[(Trifluoromethyl)sulfonyl]oxy]methyl]methylphosphinic acid, ethyl ester

A solution of dichloromethane (2 ml), pyridine (0.23 ml, 2.65 mmole), and [hydroxymethyl]methyl-phosphinic acid, ethyl ester (0.37 g, 2.65 mmole) was added dropwise to trifluoromethanesulfonic anhydride (2.65 mmole, 0.45 ml) in 2 ml of dry dichloromethane at −10°C under nitrogen. The reaction was stirred for 20 minutes at −10°C, diluted with 20 ml of dichloromethane and washed twice with 5 ml portions of water. The organic layer was dried (sodium sulfate) and concentrated *in vacuo* to yield 0.16 g of the title compound.

C) [3S-(3α, 4β)]-3-[(1,1-Dimethylethoxycarbonyl]amino]-4-methyl-2-oxo-1-azetidinyl]oxy]methyl]methyl-phosphinic acid, ethyl ester

A solution of [3S-(3α, 4β)]-3-[(2,2-dimethylethoxycarbonyl)amino]-4-methyl-2-oxo-1-hydroxyazetidine (0.155 g, 0.62 mmole) and triethylamine (0.11 ml, 0.77 mmole) in dimethylformamide (1 ml) was added to [[[(trifluoromethyl)sulfonyl]oxy]methyl]methylphosphinic acid, ethyl ester (0.21 g, .77 mmole) at −10°C under nitrogen. The reaction was stirred at 0°C for 1 hour and then concentrated *in vacuo*. The residue was dissolved in dry dichloromethane and washed successively with pH 5.5 0.5M $KH_2PO_4$, pH 3.0 $KH_2PO_4$ buffer, and water. It was then dried (sodium sulfate) and concentrated *in vacuo* to yield 0.175 g of crystalline product, melting point 84—89°C.

D) [3S-(3α, 4β)]-3-[(1,1-Dimethylethoxycarbonyl]amino]-4-methyl-2-oxo-1-azetidinyl]oxy]methyl]methyl-phosphinic acid, potassium salt

[3S-(3α, 4β)]-3-[(1,1-Dimethylethoxycarbonyl)amino]-4-methyl-2-oxo-1-azetidinyl]oxy]methyl]methyl-phosphinic acid, ethyl ester (0.175 g, 0.52 mmole) was dissolved in 1.5 ml of dry dichloromethane and cooled to −10°C under nitrogen. Bistrimethylsilylacetamide (BSA, 1.25 ml, 3.9 mmole) was added, and the

reaction was stirred for 30 minutes at 0°C. Trimethylsilylbromide (TMSBr, 0.17 ml, 1.3 mmole) was added, and the reaction was stirred at ambient temperature for 2 hours. The reaction mixture was concentrated *in vacuo*, and the residue was evaporated twice from toluene. The residue was dissolved in 2 ml of tetrahydrofuran and 2 ml of 0.5M $KH_2PO_4$ (pH 5.5) buffer and stirred at room temperature for 1 hour at pH 5. The tetrahydrofuran was removed *in vacuo* and the crude aqueous solution was washed with dichloromethane. The dichloromethane solution was concentrated *in vacuo* to yield 77 mg of azetidinyl phosphinate starting material, and the aqueous layer was chromatographed on 60 ml of HP—20 with water and then with 10% acetone-water. The appropriate fractions were combined and lyophilized to yield 52 mg of the title compound as a solid.

E) [3S-(3α, 4β)]-3-Amino-4-methyl-2-oxo-1-azetidinyl]oxy]methyl]methylphosphinic acid, trifluoroacetic acid salt

[3S-(3α, 4β)]-3-[(1,1-Dimethylethoxycarbonyl)amino]-4-methyl-2-oxo-1-azetidinyl]oxy]methyl]methylphosphinic acid, potassium salt (50 mg, 0.15 mmole) was suspended in 0.5 ml of dichloromethane and 0.5 ml of anisole at 0°C under nitrogen. Trifluoroacetic acid (1.0 ml) was added, and the reaction was stirred at 0°C for 2 hours. The solution was concentrated *in vacuo* to a residue, which was evaporated twice from toluene to give a viscous oil. This was triturated with ether to give a white solid. The NMR spectrum of this solid in $D_2O$ was consistent with that expected for the product. The $D_2O$ solution was concentrated *in vacuo* to dryness and used in the following step.

F) [3S-[3α(Z),4β]]-[[[3-[[(2-Amino-4-thiazolyl)methoxyimino)acetyl]amino]-4-methyl-2-oxo-1-azetidinyl]-oxy]methyl]methylphosphinic acid, potassium salt

(Z)-2-Amino-α-(methoxyimino)-4-thiazoleacetic acid (30.9 mg, 0.15 mmole) and 1-hydroxybenzotriazole (23.2 mg, 0.15 mmole) were dissolved in 1 ml of dimethylformamide at 0°C under nitrogen. N,N'-Dicyclohexylcarbodiimide (DCC, 31.2 mg, 0.15 mmole) was added, and the reaction was stirred at 0°C for 2 hours. [3S-(3α, 4β)]-3-Amino-4-methyl-2-oxo-1-azetidinyl]oxy]methyl]methylphosphinic acid, trifluoroacetic acid salt was dissolved in 3 ml of pH 5.5 0.5M $KH_2PO_4$ buffer, and the pH was adjusted to 5.5 with 1M potassium hydroxide. This was added to the activated ester, and the reaction was stirred at ambient temperature overnight. The reaction mixture was filtered and concentrated *in vacuo*. The residue was washed with dichloromethane, dissolved in water, and passed through 10 ml of Dowex $50K^+$ resin (0.7 meq/ml). Fractions (4 ml) were collected, and the appropriate fractions were combined and lyophilized to a residue, which was chromatographed through 30 ml of HP—20 using water to elute the product. After lyophilization, 21 mg of desired product was obtained, melting point 192—202°C, *dec.*

## Example 4

[2S-[2α,3β(Z)]]-[[[3-[[(2-Amino-4-thiazolyl)(methoxyimino)acetyl]amino]-2-methyl-4-oxo-1-azetidinyl]oxymethyl]phosphonic acid, 2-amino-2-oxoethyl ester, monopotassium salt

A) [(Hydroxy)methyl]phosphonic acid, dimethyl ester

Dimethyl phosphite (12.02 g, .11 mole) and triethylamine (1.0 g, .01 mole) were heated to 40°C under $N_2$. Paraformaldehyde (3.28 g, .11 mole) was added portionwise and the reaction mixture was heated up to 55°C. Immediate reaction occurred, and a clear solution was formed. After reaction subsided, heating was continued at 55°C for 15 minutes. The crude reaction mixture was used as is in step B.

B) [[[(Trifluoromethyl)sulfonyl]oxy]methyl]phosphonic acid, dimethyl ester

[(Hydroxy)methyl]phosphonic acid, dimethyl ester (2.10 g, 15 mmole) was dissolved in 3 ml of dry $CH_2Cl_2$ and cooled to −60°C. Triethylamine (1.52 g, 15 mmole) was added followed by the slow dropwise addition of trifluoromethanesulfonic anhydride (4.23 g, 15 mmole). The reaction was stirred until the temperature reached −25°C., $CH_2Cl_2$ (10 ml) was added, and the reaction mixture was washed with two 5 ml portions of cold water. The dichloromethane solution was dried with $Na_2SO_4$, and concentrated in vacuo to yield 3.0 g (83%) of the triflate ester.

C) [2S-[2α,3β)]-[[[-3-[[(1,1-Dimethylethoxy)carbonyl]amino]-2-methyl-4-oxo-1-azetidinyl]-oxy]-methyl-phosphonic acid, dimethyl ester

The above triflate (2.2 g, 8.1 mmole) was added dropwise to a solution of [2S-(2α,3β)-3-[[(1,1-dimethylethoxy)carbonyl]amino]-2-methyl-4-oxo-1-hydroxyazetidine (1.77 g, 8.2 mmole) and triethylamine (1.28 ml, 8.2 mmole) in 5 ml DMF at −10°C. The reaction was stirred under $N_2$ at −10°C for 4 hrs. and then concentrated in vacuo. The residue was dissolved in dry dichloromethane (150 ml) and washed successively with pH 5.5 0.5 M $KH_2PO_4$, pH 3.0 $KH_2PO_4$, and water. It was then dried ($Na_2SO_4$) and concentrated in vacuo to a volume of 4 ml. This was placed on 50 ml of SilicAR CC—7 and then filtered through with 400 ml $CH_2Cl_2$. The filtrate was concentrated to yield 1.59 g (57%) of crystalline product having M.P. 51—55°C.

D) [2S-(2α,3β)]-[[[-3-[[(1,1-Dimethylethoxy)carbonyl]amino]-2-methyl-4-oxo-1-azetidinyl]oxy]-methyl]phosphonic acid, monomethyl ester, monopotassium salt

The above azetidinyl phosphonate (1.08 g, 3.2 mmole) and thiourea (0.24 g, 3.2 mmole) were refluxed in 2 ml of dry acetonitrile under $N_2$ overnight. The solution was cooled, and the crude thiuronium salt of the product oiled out. The supernatant was decanted, and the residue was dissolved in water and passed through 70 ml of Dowex 50 in $K^+$ form (0.7 meq/ml). The product was chromatographed on 100 ml of HP—20 with water and then with 10% acetone-water. The appropriate fractions were combined and lyophilized to yield 0.53 g (48%) of product as the potassium salt.

E) [2S-(2α,3β)]-[[[3-[[(1,1-Dimethylethoxy)carbonyl]amino]-2-methyl-4-oxo-1-azetidinyl]oxy]methyl]phosphonic acid, 2-amino-2-oxoethyl ester, monopotassium salt

The above monomethyl ester, potassium salt (0.5 g, 1.4 mmole) was ion-paired with tetrabutylammonium sulfate (0.14 ml of 0.1 M in pH 5.5 buffer). Product was extracted with six 50 ml portions of $CH_2Cl_2$, dried and concentrated in vacuo to yield 0.85 g of tetrabutylammonium salt. This was dissolved in 4 ml of 1,1,1-trichloroethane. Iodoacetamide (0.57 g, 3.1 mmole) was added, and the reaction mixture was refluxed under nitrogen for 6 hrs. Volatiles were evaporated in vacuo, and the residue was passed through 50 ml of Dowex 50 2X400 .7 meq/ml in the $K^+$ form. The eluent was lyophilized and chromatographed on 70 ml of HP—20 with water and then 5% acetone-water to yield upon lyophilization 0.10 g (16%) of desired product.

F) [2S-(2α,3β)]-[[3-Amino-2-methyl-4-oxo-1-azetidinyl]oxy]methyl]phosphonic acid, mono(2-amino-2-oxoethyl)ester, trifluoroacetic acid salt

The above azetidinyl phosphonic acid, potassium salt (82 mg, .20 mmole) was suspended in 0.2 ml of $CH_2Cl_2$ and 0.2 ml of anisole at −10°C under $N_2$. Trifluoroacetic acid (0.4 ml) was added, and the reaction was stirred at 0°C for 1.5 hrs. The solution was concentrated in vacuo to a residue, which was evaporated from benzene (2X) to give a viscous oil. This was triturated with ether to give crude desired product as a white solid.

G) [2S-[2α,3β(Z)]]-[[[3-[[(2-Amino-4-thiazolyl)(methoxyimino)acetyl]amino]-2-methyl-4-oxo-1-azetidinyl]-oxy]methyl]phosphonic acid, 2-amino-2-oxoethyl ester, monopotassium salt

(Z)-(2-Amino-4-thiazolyl)methoxyimino)acetic acid (40.8 mg, .20 mmole) and 1-hydroxybenzotriazole (31.1 mg, 0.20 mmole) were dissolved in 0.4 ml of DMF at 0°C under $N_2$. N,N$^1$-Dicyclohexylcarbodiimide (DCC, 41.9 mg, 0.20 mmole) was added, and the reaction was stirred at 0°C for 2 hrs. The above crude TFA salt was dissolved in 2 ml of pH 5.5 0.5 M $KH_2PO_4$ buffer, and the pH was adjusted to 6.0 with 1 N KOH. This was added to the activated ester, and the reaction was stirred at ambient temperature overnight. The reaction mixture was filtered and concentrated in vacuo. The residue was dissolved in water and passed through 80 ml of Dowex 50K$^\oplus$ resin (0.7 meq/ml). Five ml fractions were collected, and the appropriate fractions were combined and lyophilized to a residue, which was chromatographed through 80 ml of HP—20 using water to elute the product. After lyophilization, 19 mg (19%) of desired product was obtained having m.p. 165—175° dec.

Anal. Calc'd. for $C_{13}H_{18}N_6O_8SPK \cdot 2.9H_2O$:

| | | | | | |
|---|---|---|---|---|---|
| | C, 28.87; | H, 4.44; | N, 15.54; | S, 5.90; | P, 5.70 |
| Found: | C, 29.27; | H, 3.79; | N, 14.13; | S, 5.60; | P, 5.30 |

## Example 5

### [2S-(2α,3β)]-1-[[[3-Amino-2-methyl-4-oxo-1-azetidinyl]oxy]ethyl]phosphonic acid, monomethyl ester, trifluoroacetic acid salt

A) [1-Hydroxyethyl]phosphonic acid, dimethyl ester

Dimethyl phosphite (2.34 g, 0.021 mole) acetaldehyde (0.89 g, .020 mole) and potassium fluoride (1.90 g, .020 mole) was stirred at 0°C for 2 hrs. under $N_2$. The reaction mixture was then diluted with 50 ml of $CH_2Cl_2$ and filtered to remove potassium fluoride. The filtrate was concentrated in vacuo to yield 3.02 g (98%) of product.

B) [1-[[(Trifluoromethyl)sulfonyl]oxy]ethyl]phosphonic acid, dimethyl ester

[1-Hydroxyethyl)phosphonic acid, dimethyl ester (2.11 g, 13.7 mmole) was dissolved in 3 ml of dry $CH_2Cl_2$ and cooled to −40°C under $N_2$. Triethylamine (1.39 g, 13.7 mmole) was added, followed by the slow dropwise addition of trifluoromethanesulfonic anhydride (3.87 g, 13.7 mmole). Stirring was continued until the reaction temperature reached −25°C. Dichloromethane (20 ml) was added and washed with two 4 ml portions of water. The $CH_2Cl_2$ solution was dried ($Na_2SO_4$) and concentrated in vacuo to yield 3.62 g (92%) of desired triflate.

C) [2S-(2α,3β)]-1-[[[3-[(1,1-Dimethylethoxycarbonyl)amino]-2-methyl-4-oxo-1-azetidinyl]oxy]ethyl]-phosphonic acid, dimethyl ester

The above triflate ester (2.68 g, 94 mmole) was added dropwise in two portions (30 minutes apart) at −10°C under $N_2$, to a 4 ml DMF solution of [2S-(2α,3β)]-3-[1,1-dimethylethoxycarbonyl)amino]-2-methyl-4-oxo-1-hydroxyazetidine (1.35 g, 6.3 mmole) (see SQ 28,085) and triethylamine (1.3 ml, 9.5 mmole). The solution was stirred at ambient temperature for 1 hr. then concentrated in vacuo. The residue was dissolved in dry $CH_2Cl_2$ and washed successively with pH 2.8 0.5 M $KH_2PO_4$, pH 5.5 $KH_2PO_4$ buffer and water. The $CH_2Cl_2$ was dried ($Na_2SO_4$) and concentrated in vacuo to yield 2.4 g (theory) of product as a light yellow oil.

D) [2S-(2α,3β)]-1-[[[3-[(1,1-Dimethylethoxycarbonyl)amino]-2-methyl-4-oxo-1-azetidinyl]oxy]ethyl]-phosphonic acid, monomethyl ester, monopotassium salt

The above azetidinyl phosphonate (1.35 g, 3.8 mmole) and thiourea (0.58 g, 7.7 mmole) were dissolved in 1 ml dry acetonitrile and refluxed under $N_2$ for 6 hrs. The reaction mixture was cooled and diluted with 2 ml of ether. The thiouronium salt of the product oiled out and was dissolved in 2 ml of water. This solution was passed through 100 ml of Dowex 50 $K^{\oplus}$ resin (0.7 meq/ml). Twenty ml fractions were collected, and the appropriate ones were combined and lyophilized to a residue, which was chromatographed through 100 ml of HP—20 using water. After lyophilization of the appropriate fractions, 0.89 g (68%) of desired product was obtained.

E) [2S-(2α,3β)]-1-[[[3-Amino-2-methyl-4-oxo-1-azetidinyl]oxy]ethyl]phosphonic acid, monomethyl ester, trifluoroacetic acid salt

The above azetidinyl phosphonic acid, monomethyl ester, potassium salt (0.34 g, 0.9 mmole) was suspended in 3 ml of $CH_2Cl_2$ and 3 ml of anisole at −10°C under $N_2$. Trifluoroacetic acid (6 ml) was added, and the reaction was stirred at 0°C for 2 hrs. The solution was concentrated in vacuo to a residue, which was evaporated from benzene. This was triturated with ether and dried in vacuo to give a white solid.

F) [2S-[2α,3β(Z)]]-[1-[[-3-[[(2-Amino-4-thiazolyl)(methoxyimino)acetyl]amino]-2-methyl-4-oxo-1-azetidinyl]-oxy]ethyl]phosphonic acid, monomethyl ester, monopotassium salt

(Z)-(2-Amino-4-thiazolyl)methoxyimino)acetic acid (183 mg, 0.92 mmole) and 1-hydroxybenzotriazole (139 mg, 0.92 mmole) were dissolved in 1.2 ml of DMF at 0°C (under $N_2$). N,N[1]-Dicyclohexylcarbodiimide (DCC, 188 mg, 0.92 mmole) was added, and the reaction was stirred at 0°C for 2 hrs. The above crude TFA salt was dissolved in 4 ml of pH 5.5 0.5 M $KH_2PO_4$ buffer. This was added to the activated ester, and the pH was adjusted to 4.6 with 1 M KOH. The reaction was stirred at ambient temperature overnight, filtered and concentrated in vacuo. Hydroxybenzotriazole precipitated out of the reaction mixture upon addition of ice water (5 ml pH 4.3) and was filtered. The filtrate was chromatographed through 150 ml of HP—20. Product was eluted with 5% acetone in water to yield 117 mg of desired material in the acid form after lyophilization. This was redissolved in 2 ml 0.5 M $KH_2PO_4$ buffer, and the pH was adjusted to 4.9 with 1 N KOH. This was rechromatographed through 80 ml of HP—20. The potassium salt eluted with water and was lyophilized to yield 91 mg (30%) of desired material having a m.p. 182—200°C.

Calc'd. for $C_{13}H_{19}N_5O_7PSK \cdot 1.77H_2O$:

| | | | | | |
|---|---|---|---|---|---|
| | C, 31.78; | H, 4.62; | N, 14.25; | S, 6.53; | P, 6.30 |
| Found: | C, 31.78; | H, 4.34; | N, 14.12; | S, 6.61; | P, 6.2 |

Example 6

[3S-[3α(Z),4β]]-[[3-[[(2-amino-4-thiazolyl)[(1-diphenylmethoxycarbonyl-1-methylethoxy)imino]acetyl]-amino]-4-methyl-2-oxo-1-azetidinyl]oxy]methyl]phosphonic acid, diethyl ester

By substituting (Z)-2-amino-α-[(1-diphenylmethoxycarbonyl-1-methylethoxy)imino]-4-thiazoleacetic acid for the (Z)-2-amino-α-(methoxyimino)-4-thiazoleacetic acid used in example 1(c), the above titled compound is obtained.

**Claims for the Contracting States: BE CH DE FR GB IT LI LU NL SE**

1. A compound having the formula I

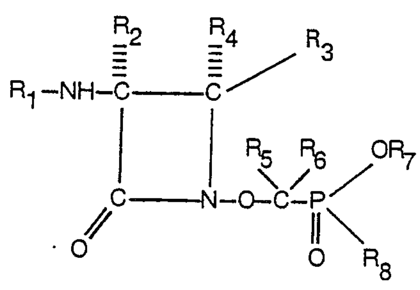

(I)

or a pharmaceutically acceptable salt thereof, wherein

$R_1$ is acyl;

$R_2$ is hydrogen or methoxy;

$R_3$ and $R_4$ are the same or different and each is hydrogen, alkyl, alkenyl, alkynyl, cycloalkyl, phenyl, substituted phenyl or a 4,5,6 or 7-membered heterocycle or one of $R_3$ and $R_4$ is hydrogen and the other is azido, halomethyl, dihalomethyl, trihalomethyl, alkoxycarbonyl, alkenyl, alkynyl, 2-phenylethenyl, 2-phenylethynyl, carboxyl,

$-CH_2X_1$, $-S-X_2$, $-O-X_2$, 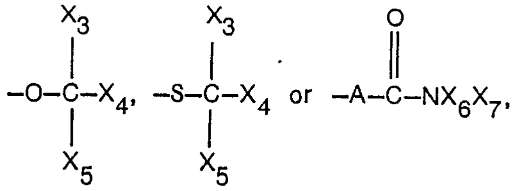 or

wherein $X_1$ is azido, amino, hydroxy, alkanoylamino, phenylcarbonylamino, (substituted phenyl)-carbonylamino, alkylsulfonyloxy, phenylsulfonyloxy, (substituted phenyl)sulfonyloxy, phenyl, substituted phenyl, cyano,

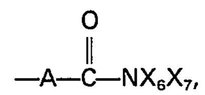

$-S-X_2$ or $-O-X_2$; $X_2$ is alkyl, substituted alkyl, phenyl, substituted phenyl, phenylalkyl, (substituted phenyl)alkyl, alkanoyl, phenylalkanoyl, (substituted phenyl)alkanoyl, phenylcarbonyl, (substituted phenyl)-carbonyl, or heteroarylcarbonyl; one of $X_3$ and $X_4$ is hydrogen and the other is hydrogen or alkyl, or $X_3$ and $X_4$ when taken together with the carbon atom to which they are attached form a cycloalkyl group;

$X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl, (substituted amino)carbonyl, or cyano;

A is $-CH=CH-$, $-(CH_2)_n-$, $-CH_2-O-$, $-CH_2-NH-$ or $-CH_2-S-CH_2-$; n is 0, 1 or 2; and

$X_6$ and $X_7$ are the same or different and each is hydrogen, alkyl, phenyl or substituted phenyl, or $X_6$ is hydrogen and $X_7$ is amino, substituted amino, acylamino or alkoxy, or $X_6$ and $X_7$ when taken together with the nitrogen atom to which they are attached form a 4, 5, 6 or 7-membered heterocycle;

$R_5$ and $R_6$ are the same or different and each is hydrogen, alkyl, alkenyl, alkynyl, phenyl, substituted phenyl, cycloalkyl or a 4, 5, 6 or 7-membered heterocycle, or $R_5$ and $R_6$ together with the carbon atom to which they are attached are cycloalkyl or a 4, 5, 6 or 7-membered heterocycle, or one of $R_5$ and $R_6$ is hydrogen and the other is azido, halomethyl, dihalomethyl, trihalomethyl, alkoxycarbonyl, alkenyl, alkynyl, 2-phenylethenyl, 2-phenylethynyl, carboxyl, $-CH_2X_1$, $-S-X_2$, $-O-X_2$, or

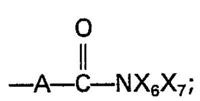

$R_7$ is hydrogen, alkyl, substituted alkyl, phenyl, substituted phenyl, 1-(ethoxycarbonyloxy)ethyl, 1,3-dihydro-3-oxo-1-isobenzofuranyl,

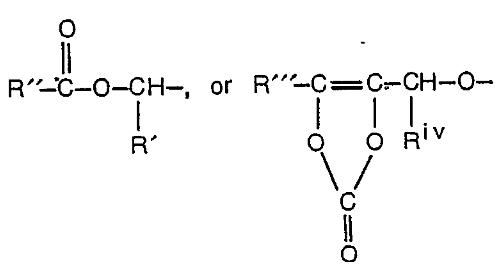
or

wherein R′ is hydrogen or alkyl, R″ is alkyl or phenyl, R‴ is hydrogen, methyl or phenyl, and $R^{iv}$ is hydrogen or together with R‴ is $-(CH_2)_3-$ or $-(CH_2)_5-$; and

$R_8$ is hydroxy, alkoxy, (substituted alkyl)oxy, phenyloxy, (substituted phenyl)oxy, alkyl, substituted alkyl, phenyl, substituted phenyl, heteroaryl, amino, substituted amino, alkylthio, (substituted alkyl)thio, phenylthio, (substituted phenyl)thio, or

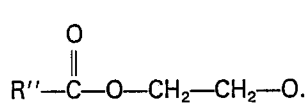

2. A compound in accordance with claim 1 wherein $R_2$ is hydrogen.

3. A compound in accordance with claim 1 wherein $R_3$ and $R_4$ are the same or different and each is hydrogen or alkyl.

4. A compound in accordance with claim 2 wherein $R_3$ and $R_4$ are the same or different and each is hydrogen or alkyl.

5. A compound in accordance with claim 1 wherein $R_5$ and $R_6$ are the same or different and each is hydrogen or alkyl.

6. A compound in accordance with claim 2 wherein $R_5$ and $R_6$ are the same or different and each is hydrogen or alkyl.

7. A compound in accordance with claim 1 wherein $R_7$ is hydrogen or alkyl and $R_8$ is hydroxy or alkoxy.

8. A compound in accordance with claim 2 wherein $R_7$ is hydrogen or alkyl and $R_8$ is hydroxy or alkoxy.

9. A compound in accordance with claim 1 wherein $R_2$ is hydrogen;
$R_3$ and $R_4$ are the same or different and each is hydrogen or alkyl;
$R_5$ and $R_6$ are the same or different and each is hydrogen or alkyl;
$R_7$ is hydrogen or alkyl; and
$R_8$ is hydroxy or alkoxy.

10. A compound in accordance with claim 1, [3S-[3α(Z),4β]]-[[[3-[[(2-amino-4-thiazolyl)(methoxyimino)-acetyl]amino]-4-methyl-2-oxo-1-azetidinyl]oxy]methyl]phosphonic acid, or a salt thereof.

11. A compound in accordance with claim 1, [3S-[3α(Z),4β]]-[[[3-[[(2-Amino-4-thiazolyl)(methoxy-imino)acetyl]amino]-4-methyl-2-oxo-1-azetidinyl]oxy]methyl]phosphonic acid, methyl ester, or a salt thereof.

12. A compound in accordance with claim 1, [3S-[3α(Z),4β]]-[[[3-[[(2-Amino-4-thiazolyl)(methoxy-imino)acetyl]amino]-4-methyl-2-oxo-1-azetidinyl]oxy]methyl]phosphonic acid, diethyl ester or a salt thereof.

13. A compound in accordance with claim 1, [3S-[3α(Z),4β]]-[[[3-[[(2-Amino-4-thiazolyl)(methoxy-imino)acetyl]amino]-4-methyl-2-oxo-1-azetidinyl]oxy]methyl]methylphosphinic acid, or a salt thereof.

14. A compound having the formula XI

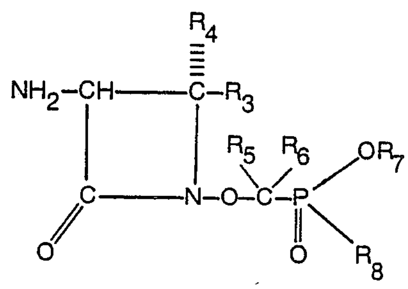

(XI)

wherein

$R_3$ and $R_4$ are the same or different and each is hydrogen, alkyl, alkenyl, alkynyl, cycloalkyl, phenyl, substituted phenyl or a 4, 5, 6 or 7-membered heterocycle or one of $R_3$ and $R_4$ is hydrogen and the other is azido, halomethyl, dihalomethyl, trihalomethyl, alkoxycarbonyl, alkenyl, alkynyl, 2-phenylethenyl, 2-phenylethynyl, carboxyl,

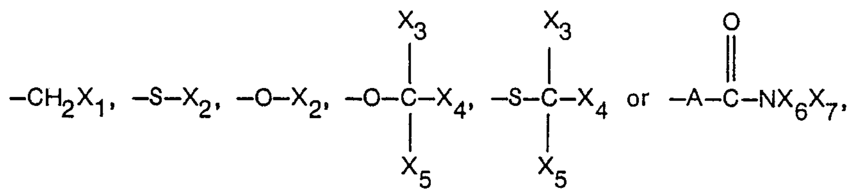

wherein $X_1$ is azido, amino, hydroxy, alkanoylamino, phenylcarbonylamino, (substituted phenyl)carbonylamino, alkylsulfonyloxy, phenylsulfonyloxy, (substituted phenyl)sulfonyloxy, phenyl, substituted phenyl, cyano,

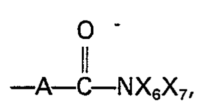

$—S—X_2$ or $—O—X_2$; $X_2$ is alkyl, substituted alkyl, phenyl, substituted phenyl, phenylalkyl, (substituted phenyl)alkyl, alkanoyl, phenylalkanoyl, (substituted phenyl)- alkanoyl, phenylcarbonyl, (substituted phenyl)carbonyl, or heteroarylcarbonyl; one of $X_3$ and $X_4$ is hydrogen and the other is hydrogen or alkyl, or $X_3$ and $X_4$ when taken together with the carbon atom to which they are attached form a cycloalkyl group; $X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl, (substituted amino)carbonyl, or cyano; A

is —CH=CH—, —$(CH_2)_n$—, —$CH_2$—O—, —$CH_2$—NH— or —$CH_2$—S—$CH_2$—; n is 0, 1 or 2; and $X_6$ and $X_7$ are the same or different and each is hydrogen, alkyl, phenyl or substituted phenyl, or $X_6$ is hydrogen and $X_7$ is amino, substituted amino, acylamino or alkoxy, or $X_6$ and $X_7$ when taken together with the nitrogen atom to which they are attached form a 4, 5, 6 or 7-membered heterocycle;

$R_5$ and $R_6$ are the same or different and each is hydrogen, alkyl, alkenyl, alkynyl, phenyl, substituted phenyl, cycloalkyl or a 4, 5, 6 or 7-membered heterocycle, or $R_5$ and $R_6$ together with the carbon atom to which they are attached are cycloalkyl or a 4, 5, 6 or 7-membered heterocycle, or one of $R_5$ and $R_6$ is hydrogen and the other is azido, halomethyl, dihalomethyl, trihalomethyl, alkoxycarbonyl, alkenyl, alkynyl, 2-phenylethenyl, 2-phenylethynyl, carboxyl, —$CH_2X_1$, —S—$X_2$, —O—$X_2$, or

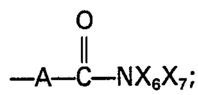

;

$R_7$ is hydrogen, alkyl, substituted alkyl, phenyl, substituted phenyl, 1-(ethoxycarbonyloxy)ethyl, 1,3-dihydro-3-oxo-1-isobenzofuranyl,

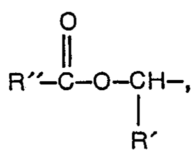

, or

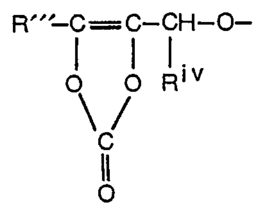

wherein R′ is hydrogen or alkyl, R″ is alkyl or phenyl, R‴ is hydrogen, methyl or phenyl, and $R^{iv}$ is hydrogen or together with R‴ is —$(CH_2)_3$— or —$(CH_2)_5$—; and

$R_8$ is hydroxy, alkoxy, (substituted alkyl)oxy, phenyloxy, (substituted phenyl)oxy, alkyl, substituted alkyl, phenyl, substituted phenyl, heteroaryl, amino, substituted amino, alkylthio, (substituted alkyl)thio, phenylthio, (substituted phenyl)thio,

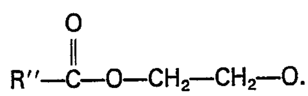

.

15. A compound of the formula I according to claim 1 for use in the treatment of bacterial infections.

16. Pharmaceutical composition comprising a compound of the formula I according to claim 1 and a physiologically acceptable vehicle.

**Claims for the Contracting State: AT**

1. A process for preparing a compound having the formula I

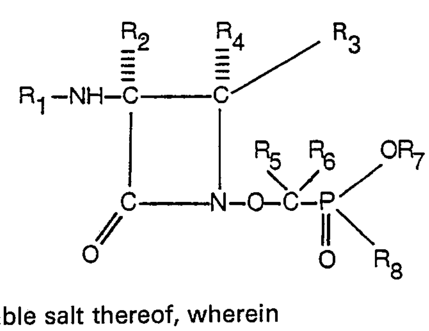

(I)

or a pharmaceutically acceptable salt thereof, wherein

$R_1$ is acyl;

$R_2$ is hydrogen or methoxy;

$R_3$ and $R_4$ are the same or different and each is hydrogen, alkyl, alkenyl, alkynyl, cycloalkyl, phenyl, substituted phenyl or a 4, 5, 6 or 7-membered heterocycle or one of $R_3$ and $R_4$ is hydrogen and the other is

azido, halomethyl, dihalomethyl, trihalomethyl, alkoxycarbonyl, alkenyl, alkynyl, 2-phenylethenyl, 2-phenylethynyl, carboxyl,

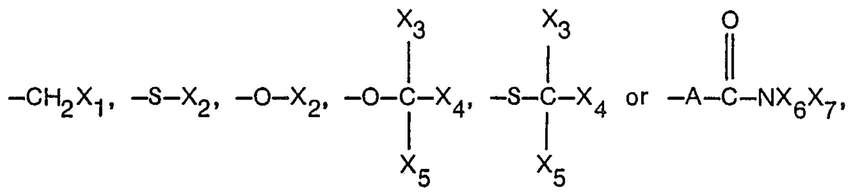

wherein $X_1$ is azido, amino, hydroxy, alkanoylamino, phenylcarbonylamino, (substituted phenyl)carbonylamino, alkylsulfonyloxy, phenylsulfonyloxy, (substituted phenyl)sulfonyloxy, phenyl, substituted phenyl, cyano,

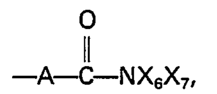

$-S-X_2$ or $-O-X_2$; $X_2$ is alkyl, substituted alkyl, phenyl, substituted phenyl, phenylalkyl, (substituted phenyl)alkyl, alkanoyl, phenylalkanoyl, (substituted phenyl)alkanoyl, phenylcarbonyl, (substituted phenyl)carbonyl, or heteroarylcarbonyl; one of $X_3$ and $X_4$ is hydrogen and the other is hydrogen or alkyl, or $X_3$ and $X_4$ when taken together with the carbon atom to which they are attached form a cycloalkyl group; $X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl, (substituted amino)carbonyl, or cyano; A is $-CH=CH-$, $-(CH_2)_n-$, $-CH_2-O-$, $-CH_2-NH-$ or $-CH_2-S-CH_2-$; n is 0, 1 or 2; and $X_6$ and $X_7$ are the same or different and each is hydrogen, alkyl, phenyl or substituted phenyl, or $X_6$ is hydrogen and $X_7$ is amino, substituted amino, acylamino or alkoxy, or $X_6$ and $X_7$ when taken together with the nitrogen atom to which they are attached form a 4, 5, 6 or 7-membered heterocycle;

$R_5$ and $R_6$ are the same or different and each is hydrogen, alkyl, alkenyl, alkynyl, phenyl, substituted phenyl, cycloalkyl or a 4, 5, 6 or 7-membered heterocycle, or $R_5$ and $R_6$ together with the carbon atom to which they are attached are cycloalkyl or a 4, 5, 6 or 7-membered heterocycle, or one of $R_5$ and $R_6$ is hydrogen and the other is azido, halomethyl, dihalomethyl, trihalomethyl, alkoxycarbonyl, alkenyl, alkynyl, 2-phenylethenyl, 2-phenylethynyl, carboxyl, $-CH_2X_1$, $-S-X_2$, $-O-X_2$, or

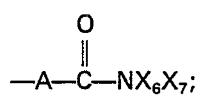

$R_7$ is hydrogen, alkyl, substituted alkyl, phenyl, substituted phenyl, 1-(ethoxycarbonyloxy)ethyl, 1,3-dihydro-3-oxo-1-isobenzofuranyl,

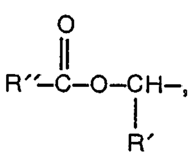

or

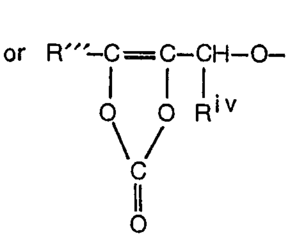

wherein R′ is hydrogen or alkyl, R″ is alkyl or phenyl, R‴ is hydrogen, methyl or phenyl, and $R^{iv}$ is hydrogen or together with R‴ is $-(CH_2)_3-$ or $-(CH_2)_5-$; and

$R_8$ is hydroxy, alkoxy, (substituted alkyl)oxy, phenyloxy, (substituted phenyl)oxy, alkyl, substituted alkyl, phenyl, substituted phenyl, heteroaryl, amino, substituted amino, alkylthio, (substituted alkyl)thio, phenylthio, (substituted phenyl)thio, or

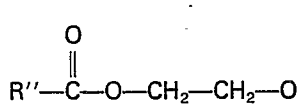

characterized in that a compound having the formula

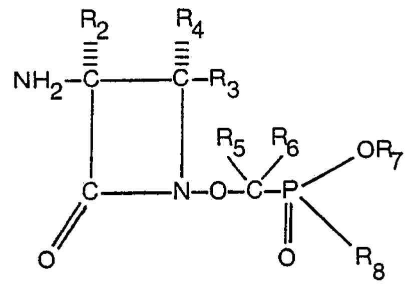

is acylated according to known acylation techniques e.g. by reaction with a carboxylic acid ($R_1$—OH) or corresponding carboxylic acid halide or carboxylic acid anhydride, and the product obtained is optionally converted into a pharmaceutically acceptable salt thereof.

2. A process in accordance with claim 1 wherein $R_2$ is hydrogen.

3. A process in accordance with claim 1 wherein $R_3$ and $R_4$ are the same or different and each is hydrogen or alkyl.

4. A process in accordance with claim 2 wherein $R_3$ and $R_4$ are the same or different and each is hydrogen or alkyl.

5. A process in accordance with claim 1 wherein $R_5$ and $R_6$ are the same or different and each is hydrogen or alkyl.

6. A process in accordance with claim 2 wherein $R_5$ and $R_6$ are the same or different and each is hydrogen or alkyl.

7. A process in accordance with claim 1 wherein $R_7$ is hydrogen or alkyl and $R_8$ is hydroxy or alkoxy.

8. A process in accordance with claim 2 wherein $R_7$ is hydrogen or alkyl and $R_8$ is hydroxy or alkoxy.

9. A process in accordance with claim 1 wherein $R_2$ is hydrogen;
$R_3$ and $R_4$ are the same or different and each is hydrogen or alkyl;
$R_5$ and $R_6$ are the same or different and each is hydrogen or alkyl;
$R_7$ is hydrogen or alkyl; and
$R_8$ is hydroxy or alkoxy.

10. A process in accordance with claim 1, for the production of [3S-[3α(Z),4β]]-[[[3-[[(2-amino-4-thiazolyl)(methoxyimino)acetyl]amino]-4-methyl-2-oxo-1-azetidinyl]oxy]methyl]phosphonic acid, or a salt thereof.

11. A process in accordance with claim 1, for the production of [3S-[3α(Z),4β]]-[[[3-[[(2-Amino-4-thiazolyl)(methoxyimino)acetyl]amino]-4-methyl-2-oxo-1-azetidinyl]oxy]methyl]phosphonic acid, methyl ester, or a salt thereof.

12. A process in accordance with claim 1, for the production of [3S-[3α(Z),4β]]-[[[3-[[(2-Amino-4-thiazolyl)(methoxyimino)acetyl]amino]-4-methyl-2-oxo-1-azetidinyl]oxy]methyl]phosphonic acid, diethyl ester or a salt thereof.

13. A process in accordance with claim 1, for the production of [3S-[3α(Z),4β]]-[[[3-[[(2-Amino-4-thiazolyl)(methoxyimino)acetyl]amino]-4-methyl-2-oxo-1-azetidinyl]oxy]methyl]methylphosphinic acid, or a salt thereof.

14. A process for preparing a compound having the formula XI

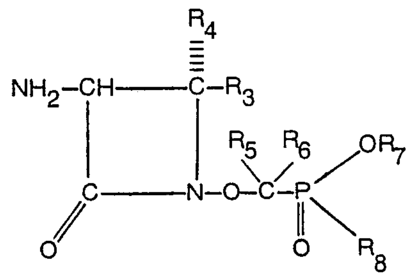

(XI)

wherein

$R_3$ and $R_4$ are the same or different and each is hydrogen, alkyl, alkenyl, alkynyl, cycloalkyl, phenyl, substituted phenyl or a 4, 5, 6 or 7-membered heterocycle or one of $R_3$ and $R_4$ is hydrogen and the other is azido, halomethyl, dihalomethyl, trihalomethyl, alkoxycarbonyl, alkenyl, alkynyl, 2-phenylethenyl, 2-phenylethynyl, carboxyl,

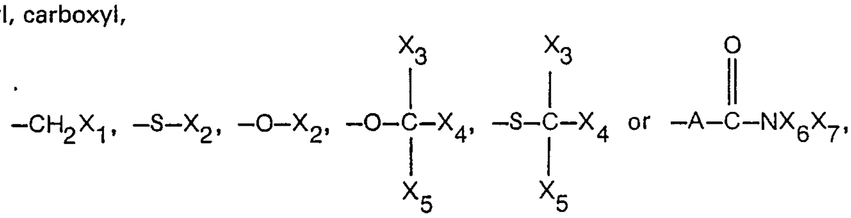

wherein $X_1$ is azido, amino, hydroxy, alkanoylamino, phenylcarbonylamino, (substituted phenyl)carbonylamino, alkylsulfonyloxy, phenylsulfonyloxy, (substituted phenyl)sulfonyloxy, phenyl, substituted phenyl, cyano,

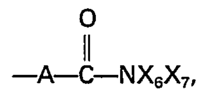

$—S—X_2$ or $—O—X_2$; $X_2$ is alkyl, substituted alkyl, phenyl, substituted phenyl, phenylalkyl, (substituted phenyl)alkyl, alkanoyl, phenylalkanoyl, (substituted phenyl)alkanoyl, phenylcarbonyl, (substituted phenyl)-carbonyl, or heteroarylcarbonyl; one of $X_3$ and $X_4$ is hydrogen and the other is hydrogen or alkyl, or $X_3$ and $X_4$ when taken together with the carbon atom to which they are attached form a cycloalkyl group; $X_5$ is formyl, alkanoyl, phenylcarbonyl, (substituted phenyl)carbonyl, phenylalkylcarbonyl, (substituted phenyl)-alkylcarbonyl, carboxyl, alkoxycarbonyl, aminocarbonyl, (substituted amino)carbonyl, or cyano; A is $—CH{=}CH—$, $—(CH_2)_n—$, $—CH_2—O—$, $—CH_2—NH—$ or $—CH_2—S—CH_2—$; n is 0, 1 or 2; and $X_6$ and $X_7$ are the same or different and each is hydrogen, alkyl, phenyl or substituted phenyl, or $X_6$ is hydrogen and $X_7$ is amino, substituted amino, acylamino or alkoxy, or $X_6$ and $X_7$ when taken together with the nitrogen atom to which they are attached form a 4, 5, 6 or 7-membered heterocycle;

$R_5$ and $R_6$ are the same or different and each is hydrogen, alkyl, alkenyl, alkynyl, phenyl, substituted phenyl, cycloalkyl or a 4, 5, 6 or 7-membered heterocycle, or $R_5$ and $R_6$ together with the carbon atom to which they are attached are cycloalkyl or a 4, 5, 6 or 7-membered heterocycle, or one of $R_5$ and $R_6$ is hydrogen and the other is azido, halomethyl, dihalomethyl, trihalomethyl, alkoxycarbonyl, alkenyl, alkynyl, 2-phenylethenyl, 2-phenylethynyl, carboxyl, $—CH_2X_1$, $—S—X_2$, $—O—X_2$, or

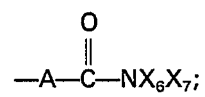

$R_7$ is hydrogen, alkyl, substituted alkyl, phenyl, substituted phenyl, 1-(ethoxycarbonyloxy)ethyl, 1,3-dihydro-3-oxo-1-isobenzofuranyl,

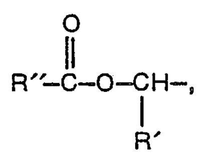

or

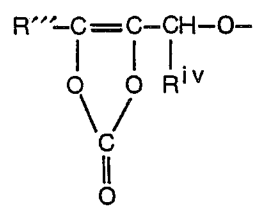

wherein R′ is hydrogen or alkyl, R″ is alkyl or phenyl, R‴ is hydrogen, methyl or phenyl, and $R^{iv}$ is hydrogen or together with R‴ is $—(CH_2)_3—$ or $—(CH_2)_5—$; and

$R_8$ is hydroxy, alkoxy, (substituted alkyl)oxy, phenyloxy, (substituted phenyl)oxy, alkyl, substituted alkyl, phenyl, substituted phenyl, heteroaryl, amino, substituted amino, alkylthio, (substituted alkyl)thio, phenylthio, (substituted phenyl)thio,

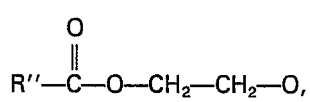

characterized in that a compound having the formula VIII

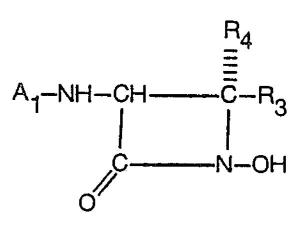

VIII

is alkylated with an activated form of a compound having the formula IX

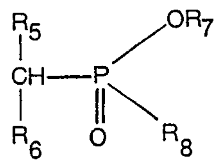

IX

and the resulting product having the formula X

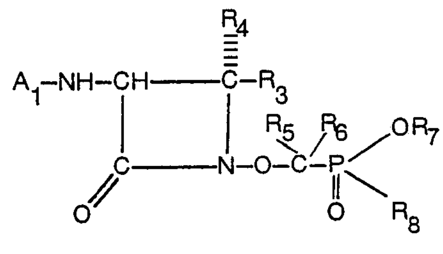

X

is converted to a compound having the formula XI by deprotection of the 3-amino substituent.

15. A method for preparing a pharmaceutical composition comprising combining a compound of the formula I obtained according to Claim 1 with a physiologically acceptable vehicle.

**Patentansprüche für die Vertragsstaaten: BE CH DE FR GB IT LI LU NL SE**

1. Eine verbindung der Formel I

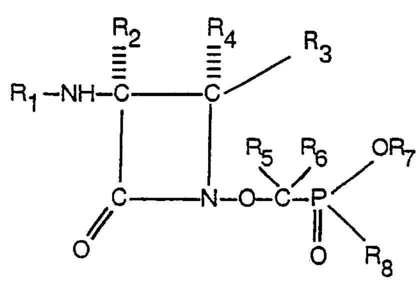

( I )

oder ein pharmazeutisch verträgliches Salz davon, in der

$R_1$ Acyl ist;

$R_2$ Wasserstoff oder Methoxy ist;

$R_3$ und $R_4$ gleich oder verschieden sind und jeweils Wasserstoff, Alkyl, Alkenyl, Alkinyl, Cycloalkyl, Phenyl, substituiertes Phenyl oder einen 4,5,6- oder 7-gliedrigen Heterocyclus bedeuten oder einer der Reste $R_3$ und $R_4$ ein Wasserstoffatom ist und der andere Azido, Halogenmethyl, Dihalogenmethyl, Trihalogenmethyl, Alkoxycarbonyl, Alkenyl, Alkinyl, 2-Phenyläthenyl, 2-Phenyläthinyl, Carboxyl oder einen der Reste

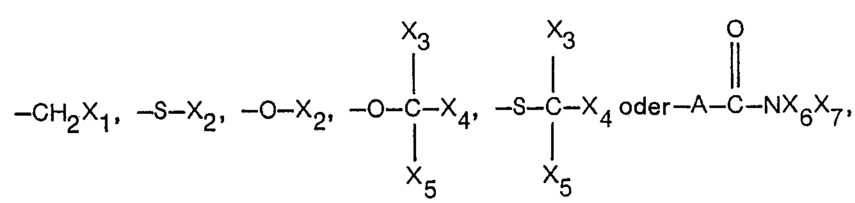

bedeutet, wobei

$X_1$ Azido, Amino, Hydroxy, Alkanoylamino, Phenylcarbonylamino, (substituiertes Phenyl)-carbonylamino, Alkylsulfonyloxy, Phenylsulfonyloxy, (substituiertes Phenyl)-sulfonyloxy, Phenyl, subtituiertes Phenyl, Cyano,

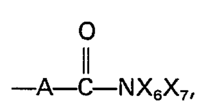

—S—$X_2$ oder —O—$X_2$ bedeutet;

$X_2$ Alkyl, substituiertes Alkyl, Phenyl, substituiertes Phenyl, Phenylalkyl, (substituiertes Phenyl)-alkyl, Alkanoyl, Phenylalkanoyl, (substituiertes Phenyl)-alkanoyl, Phenylcarbonyl, (substituiertes Phenyl)-carbonyl oder Heteroarylcarbonyl darstellt;

einer der Reste $X_3$ und $X_4$ Wasserstoff ist und der andere Wasserstoff oder Alkyl bedeutet, oder $X_3$ und $X_4$ zusammen mit dem Kohlenstoffatomen, an das sie gebunden sind, einen Cycloalkylrest bilden;

$X_5$ Formyl, Alkanoyl, Phenylcarbonyl, (substituiertes Phenyl)-carbonyl, Phenylalkylcarbonyl, (substituiertes Phenyl)-alkylcarbonyl, Carboxyl, Alkoxycarbonyl, Aminocarbonyl, (substituiertes Amino)-carbonyl oder Cyano darstellt;

A eine Gruppen —CH=CH—, $(CH_2)_n$—, —$CH_2$—O—, —$CH_2$—NH— oder —$CH_2$—S—$CH_2$— bedeutet;

n 0, 1 oder 2 ist; und

$X_6$ und $X_7$ gleich oder verschieden sind und jeweils Wasserstoff, Alkyl, Phenyl oder substituiertes Phenyl bedeuteten, oder $X_6$ Wasserstoff ist und $X_7$ Amino, substituiertes Amino, Acylamino oder Alkoxy darstellt, oder $X_6$ und $X_7$ zusammen mit dem Stickstoffatom, an das sie gebunden sind, einen 4-, 5-, 6-oder 7-gliedrigen Heterocyclus bilden,

$R_5$ und $R_6$ gleich oder verschieden sind und jeweils Wasserstoff, Alkyl, Alkenyl, Alkinyl, Phenyl, substituiertes Phenyl, Cycloalkyl oder einen 4-, 5-, 6-oder 7-gliedrigen Heterocyclus bilden, oder $R_5$ und $R_6$ zusammen mit dem Kohlenstoffatom, an das sie gebunden sind, Cycloalkyl oder einen 4-, 5-, 6-oder 7-gliedrigen Heterocyclus bedeuten oder einer der Reste $R_5$ und $R_6$ ein Wasserstoffatom ist und der andere Azido, Halogenmethyl, Dihalogenmethyl, Trihalogenmethyl, Alkoxycarbonyl, Alkenyl, Alkinyl, 2-Phenyläthenyl, 2-Phenyläthinyl, Carboxyl, oder einen der Reste —$CH_2X_1$, —S—$X_2$, —O—$X_2$ oder

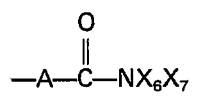

bedeutet;

$R_7$ Wasserstoff, Alkyl, substituiertes Alkyl, Phenyl, substituiertes Phenyl, 1-(Äthoxycarbonyloxy)-äthyl, 1,3-Dihydro-3-oxo-1-isobenzofuranyl, oder eine der Gruppen

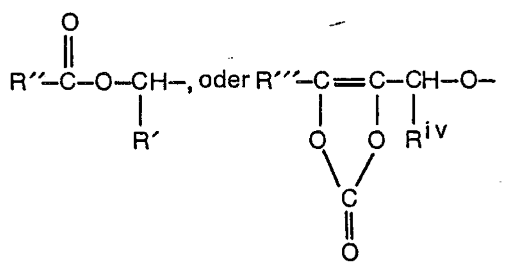

bedeutet,

wobei R′ Wasserstoff oder Alkyl ist, R″ Alkyl oder Phenyl bedeutet, R‴ Wasserstoff, Methyl oder Phenyl darstellt und $R^{iv}$ Wasserstoff ist oder zusammen mit R‴ —$(CH_2)_3$— oder —$(CH_2)_5$— bedeutet; und

$R_8$ Hydroxy, Alkoxy, (substituiertes Alkyl)-oxy, Phenyloxy, (substituiertes Phenyl)-oxy, Alkyl, substituiertes Alkyl, Phenyl, substituiertes Phenyl, Heteroaryl, Amino, substituiertes Amino, Alkylthio, (substituiertes Alkyl)-thio, Phenylthio, (substituiertes Phenyl)-thio oder

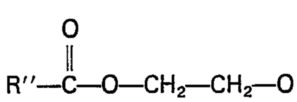

bedeutet.

2. Verbindung nach Anspruch 1, in der $R_2$ Wasserstoff ist.

3. Verbindung nach Anspruch 1, in der $R_3$ und $R_4$ gleich oder verschieden sind und jeweils Wasserstoff oder Alkyl bedeuten.

4. Verbindung nach Anspruch 2, in der $R_3$ und $R_4$ gleich oder verschieden sind und jeweils Wasserstoff oder Alkyl bedeuten.

5. Verbindung nach Anspruch 1, in der $R_5$ und $R_6$ gleich oder verschieden sind und jeweils Wasserstoff oder Alkyl bedeuten.

6. Verbindung nach Anspruch 2, in der $R_5$ und $R_6$ gleich oder verschieden sind und jeweils Wasserstoff oder Alkyl bedeuten.

7. Verbindung nach Anspruch 1, in der $R_7$ Wasserstoff oder Alkyl und $R_8$ Hydroxy oder Alkoxy bedeuten.

8. Verbindung nach Anspruch 2, in der $R_7$ Wasserstoff oder Alkyl und $R_8$ Hydroxy oder Alkoxy bedeuten.

9. Verbindung nach Anspruch 1, in der $R_2$ Wasserstoff ist;
$R_3$ und $R_4$ gleich oder veschieden sind und jeweils Wasserstoff oder Alkyl bedeuten;
$R_5$ und $R_6$ gleich oder verschieden sind und jeweils Wasserstoff oder Alkyl bedeuten;
$R_7$ Wasserstoff oder Alkyl ist; und
$R_8$ Hydroxy oder Alkoxy darstellt.

10. Verbindung nach Anspruch 1, [3S-[3α(Z),4β]]-[[[3-[[(2-Amino-4-thiazolyl)-(methoxyimino)-acetyl]-amino]-4-methyl-2-oxo-1-azetidinyl]-oxy]-methyl]-phosphonsäure oder ein Salz davon.

11. Verbindung nach Anspruch 1, [3S-[3α(Z),4β]]-[[[3-[[(2-Amino-4-thiazolyl)-(methoxyimino)-acetyl]-amino-4-methyl-2-oxo-1-azetidinyl]-oxy]-methyl]-phosphonsäure-methylester oder ein Salz davon.

12. Verbindung nach Anspruch 1, [3S-[3α(Z),4β]]-[[[3-[[(2-Amino-4-thiazolyl)-(methoxyimino)-acetyl]-amino-4-methyl-2-oxo-1-azetidinyl]-oxy]-methyl]-phosphonsäure-diäthylester oder ein Salz davon.

13. Verbindung nach Anspruch 1, [3S-[3α(Z),4β]]-[[[3-[[(2-Amino-4-thiazolyl)-(methoxyimino)-acetyl]-amino-4-methyl-2-oxo-1-azetidinyl]-oxy]-methyl]-phosphonsäure oder ein Salz davon.

14. Eine Verbindung der Formel XI

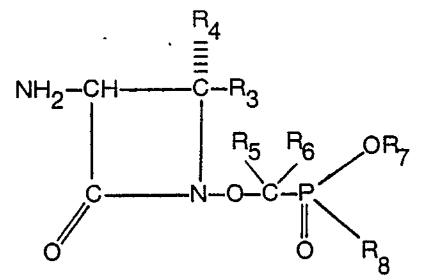

(XI)

in der
$R_3$ und $R_4$ gleich oder verschieden sind und jeweils Wasserstoff, Alkyl, Alkenyl, Alkinyl, Cycloalkyl, Phenyl, substituiertes Phenyl oder einen 4,5,6- oder 7-gliedrigen Heterocyclus bedeuten oder einer der Reste $R_3$ und $R_4$ ein Wasserstoffatom ist und der andere Azido, Halogenmethyl, Dihalogenmethyl, Trihalogenmethyl, Alkoxycarbonyl, Alkenyl, Alkinyl, 2-Phenyläthenyl, 2-Phenyläthinyl, Carboxyl oder einen der Reste

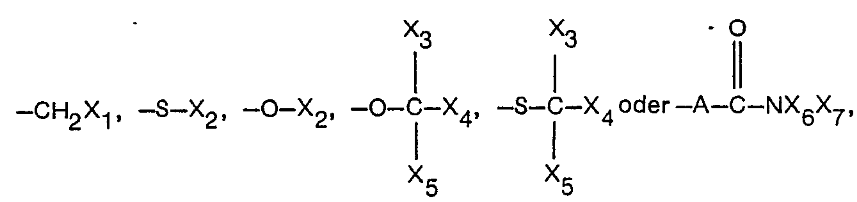

bedeutet, wobei
$X_1$ Azido, Amino, Hydroxy, Alkanoylamino, Phenylcarbonylamino, (substituiertes Phenyl)-carbonylamino, Alkylsulfonyloxy, Phenylsulfonyloxy, (substituiertes Phenyl)-sulfonyloxy, Phenyl, substituiertes Phenyl, Cyano,

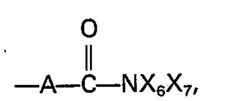

$—S—X_2$ oder $—O—X_2$ bedeutet;
$X_2$ Alkyl, substituiertes Alkyl, Phenyl, substituiertes Phenyl, Phenylalkyl, (substituiertes Phenyl)-alkyl, Alkanoyl, Phenylalkanoyl, (substituiertes Phenyl)-alkanoyl, Phenylcarbonyl, (substituiertes Phenyl)-carbonyl oder Heteroarylcarbonyl darstellt;
einer der Reste $X_3$ und $X_4$ Wasserstoff ist und der andere Wasserstoff oder Alkyl bedeutet, oder $X_3$ und $X_4$ zusammen mit dem Kohlenstoffatom, an das sie gebunden sind, einen Cycloalkylrest bilden;
$X_5$ Formyl, Alkanoyl, Phenylcarbonyl, (substituiertes Phenyl)-carbonyl, Phenylalkylcarbonyl, (substituiertes Phenyl)-alkylcarbonyl, Carboxyl, Alkoxycarbonyl, Aminocarbonyl, (substituiertes Amino)-carbonyl oder Cyano darstellt;
A eine der Gruppen $—CH{=}CH—$, $—(CH_2)_n—$, $—CH_2—O—$, $—CH_2—NH-$ oder $—CH_2—S—CH_2—$ bedeutet;
n 0, 1 oder 2 ist; und
$X_6$ und $X_7$ gleich oder verschieden sind und jeweils Wasserstoff, Alkyl, Phenyl oder substituiertes Phenyl bedeuten, oder $X_6$ Wasserstoff ist und $X_7$ Amino, substituiertes Amino, Acylamino oder Alkoxy darstellt, oder $X_6$ und $X_7$ zusammen mit dem Stickstoffatom, an das sie gebunden sind, einen 4, 5, 6 oder 7-gliedrigen Heterocyclus bilden,

$R_5$ und $R_6$ gleich oder verschieden sind und jeweils Wasserstoff, Alkyl, Alkenyl, Alkinyl, Phenyl, substituiertes Phenyl, Cycloalkyl oder einen 4-, 5-, 6- oder 7-gliedrigen Heterocyclus bilden, oder $R_5$ und $R_6$ zusammen mit dem Kohlenstoffatom, an das sie gebunden sind, Cycloalkyl oder einen 4-, 5-, 6- oder 7-gliedrigen Heterocyclus bedeuten oder einer der Reste $R_5$ und $R_6$ ein Wasserstoffatom ist und der andere Azido, Halogenmethyl, Dihalogenmethyl, Trihalogenmethyl, Alkoxycarbonyl, Alkenyl, Alkinyl, 2-Phenyläthenyl, 2-Phenyläthinyl, Carboxyl, oder einen der Reste $—CH_2X_1$, $—S—X_2$, $—O—X_2$ oder

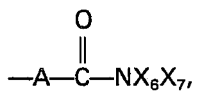

bedeutet;

$R_1$ Wasserstoff, Alkyl, substituiertes Alkyl, Phenyl, substituiertes Phenyl, 1-(Äthoxycarbonyloxy)-äthyl, 1,3-Dihydro-3-oxo-1-isobenzofuranyl, oder eine der Gruppen

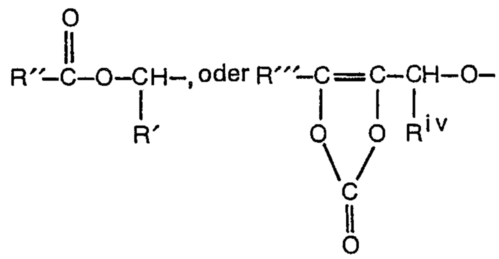

bedeutet,

wobei R′ Wasserstoff oder Alkyl ist, R″ Alkyl oder Phenyl bedeutet, R‴ Wasserstoff, Methyl oder Phenyl darstellt und $R^{iv}$ Wasserstoff ist oder zusammen mit R‴ $—(CH_2)_3—$ oder $—(CH_2)_5—$ bedeutet; und

$R_8$ Hydroxy, Alkoxy, (substituiertes Alkyl)-oxy, Phenyloxy, (substituiertes Phenyl)-oxy, Alkyl, substituiertes Alkyl, Phenyl, substituiertes Phenyl, Heteroaryl, Amino, substituiertes Amino, Alkylthio, (substituiertes Alkyl)-thio, Phenylthio, (substituiertes Phenyl)-thio oder

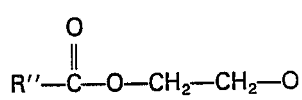

bedeutet.

15. Eine Verbindung der Formel I nach Anspruch 1 zur Verwendung bei der Behandlung von bakteriellen Infektionen.

16. Arzneimittel, umfassend eine Verbindung der Formel I nach Anspruch 1 und einen physiologisch verträglichen Träger.

**Patentansprüche für den Vertragsstaat: AT**

1. Ein Verfahren zur Herstellung einer Verbindung der Formel I

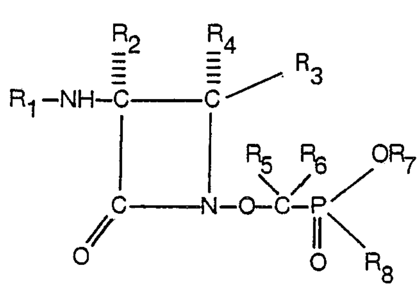

(I)

oder ein pharmazeutisch verträglichen Salzes davon, in der

$R_1$ Acyl ist;

$R_2$ Wasserstoff oder Methoxy ist;

$R_3$ und $R_4$ gleich oder verschieden sind und jeweils Wasserstoff, Alkyl, Alkenyl, Alkinyl, Cycloalkyl, Phenyl, substituiertes Phenyl oder einen 4,5,6- oder 7-gliedrigen Heterocyclus bedeuten oder einer der Reste $R_3$ und $R_4$ ein Wasserstoffatom ist und der andere Azido, Halogenmethyl, Dihalogenmethyl,

Trihalogenmethyl, Alkoxycarbonyl, Alkenyl, Alkinyl, 2-Phenyläthenyl, 2-Phenyläthinyl, Carboxyl oder einen der Reste

$-CH_2X_1$, $-S-X_2$, $-O-X_2$, 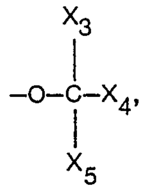, 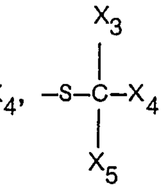 oder 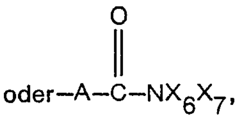,

bedeutet, wobei

$X_1$ Azido, Amino, Hydroxy, Alkanoylamino, Phenylcarbonylamino, (substituiertes Phenyl)-carbonylamino, Alkylsulfonyloxy, Phenylsulfonyloxy, (substituiertes Phenyl)-sulfonyloxy, Phenyl, subtituiertes Phenyl, Cyano,

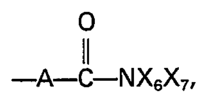

$-S-X_2$ oder $-O-X_2$ bedeutet;

$X_2$ Alkyl, substituiertes Alkyl, Phenyl, substituiertes Phenyl, Phenylalkyl, (substituiertes Phenyl)-alkyl, Alkanoyl, Phenylalkanoyl, (substituiertes Phenyl)-alkanoyl, Phenylcarbonyl, (substituiertes Phenyl)-carbonyl oder Heteroarylcarbonyl darstellt;

einer der Reste $X_3$ und $X_4$ Wasserstoff ist und der andere Wasserstoff oder Alkyl bedeutet, oder $X_3$ und $X_4$ zusammen mit dem Kohlenstoffatom, an das sie gebunden sind, einen Cycloalkylrest bilden;

$X_5$ Formyl, Alkanoyl, Phenylcarbonyl, (substituiertes Phenyl)-carbonyl, Phenylalkylcarbonyl, (substituiertes Phenyl)-alkylcarbonyl, Carboxyl, Alkoxycarbonyl, Aminocarbonyl, (substituiertes Amino)-carbonyl oder Cyano darstellt;

A eine Gruppen $-CH=CH-$, $-(CH_2)_n-$, $-CH_2-O-$, $-CH_2-NH-$ oder $-CH_2-S-CH_2-$ bedeutet;

n 0, 1 oder 2 ist; und

$X_6$ und $X_7$ gleich oder verschieden sind und jeweils Wasserstoff, Alkyl, Phenyl oder substituiertes Phenyl bedeutet, oder $X_6$ Wasserstoff ist und $X_7$ Amino, substituiertes Amino, Acylamino oder Alkoxy darstellt, oder $X_6$ und $X_7$ zusammen mit dem Stickstoffatom, an das sie gebunden sind, einen 4-, 5-, 6-oder 7-gliedrigen Heterocyclus bilden,

$R_5$ und $R_6$ gleich oder verschieden sind und jeweils Wasserstoff, Alkyl, Alkenyl, Alkinyl, Phenyl, substituiertes Phenyl, Cycloalkyl oder einen 4-, 5-, 6-oder 7-gliedrigen Heterocyclus bilden, oder $R_5$ und $R_6$ zusammen mit dem Kohlenstoffatom, an das sie gebunden sind, Cycloalkyl oder einen 4-, 5-, 6-oder 7-gliedrigen Heterocyclus bedeuten oder einer der Reste $R_5$ und $R_6$ ein Wasserstoffatom ist und der andere Azido, Halogenmethyl, Dihalogenmethyl, Trihalogenmethyl, Alkoxycarbonyl, Alkenyl, Alkinyl, 2-Phenyläthenyl, 2-Phenyläthinyl, Carboxyl, oder einen der Reste $-CH_2X_1$, $-S-X_2$, $-O-X_2$ oder

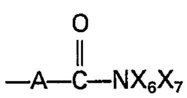

bedeutet;

$R_7$ Wasserstoff, Alkyl, substituiertes Alkyl, Phenyl, substituiertes Phenyl, 1-(Äthoxycarbonyloxy)-äthyl, 1,3-Dihydro-3-oxo-1-isobenzofuranyl, oder eine der Gruppen

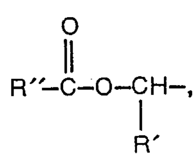, oder 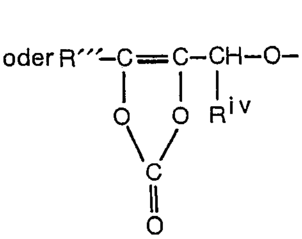

bedeutet,

wobei R′ Wasserstoff oder Alkyl ist, R″ Alkyl oder Phenyl bedeutet, R‴ Wasserstoff, Methyl oder Phenyl darstellt und $R^{iv}$ Wasserstoff ist oder zusammen mit R‴ $-(CH_2)_3-$ oder $-(CH_2)_5-$ bedeutet; und

$R_8$ Hydroxy, Alkoxy, (substituiertes Alkyl)-oxy, Phenyloxy, (substituiertes Phenyl)-oxy, Alkyl, substituiertes Alkyl, Phenyl, substituiertes Phenyl, Heteroaryl, Amino, substituiertes Amino, Alkylthio,

(substituiertes Alkyl)-thio, Phenylthio, (substituiertes Phenyl)-thio oder

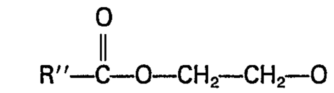

bedeutet, dadurch gekennzeichnet, daß eine Verbindung der Formel

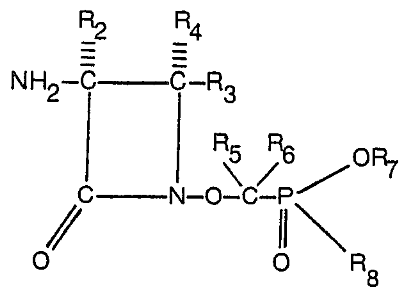

nach bekannten Acylierungsverfahren, z.B. durch Umsetzung mit einer Carbonsäure ($R_1$—OH) oder einem entsprechenden Carbonsäurehalogenid oder Carbonsäureanhydrid acyliert wird und gegebenenfalls das erhaltene Endprodukt in ein pharmazeutisch verträgliches Salz davon umgewandelt wird.

2. Verfahren nach Anspruch 1, wobei $R_2$ Wasserstoff ist.

3. Verfahren nach Anspruch 1, wobei $R_3$ und $R_4$ gleich oder verschieden sind und jeweils Wasserstoff oder Alkyl bedeuten.

4. Verfahren nach Anspruch 2, wobei $R_3$ und $R_4$ gleich oder verschieden sind und jeweils Wasserstoff oder Alkyl bedeuten.

5. Verfahren nach Anspruch 1, wobei $R_5$ und $R_6$ gleich oder verschieden sind und jeweils Wasserstoff oder Alkyl bedeuten.

6. Verfahren nach Anspruch 2, wobei $R_5$ und $R_6$ gleich oder verschieden sind und jeweils Wasserstoff oder Alkyl bedeuten.

7. Verfahren nach Anspruch 1, wobei $R_7$ Wasserstoff oder Alkyl und $R_8$ Hydroxy oder Alkoxy bedeuten.

8. Verfahren nach Anspruch 2, wobei $R_7$ Wasserstoff oder Alkyl und $R_8$ Hydroxy oder Alkoxy bedeuten.

9. Verfahren nach Anspruch 1, wobei $R_2$ Wasserstoff ist;
$R_3$ und $R_4$ gleich oder verschieden sind und jeweils Wasserstoff oder Alkyl bedeuten;
$R_5$ und $R_6$ gleich oder verschieden sind und jeweils Wasserstoff oder Alkyl bedeuten;
$R_7$ Wasserstoff oder Alkyl ist; und
$R_8$ Hydroxy oder Alkoxy darstellt.

10. Verfarhen nach Anspruch 1 zur Herstellung von [3S-[3α(Z),4β]]-[[[3-[[(2-Amino-4-thiazolyl)-(methoxyimino)-acetyl]-amino]-4-methyl-2-oxo-1-azetidinyl]-oxy]-methyl]-phosphonsäure oder einem Salz davon.

11. Verfahren nach Anspruch 1 zur Herstellung von [3S-[3α(Z),4β]]-[[[3-[[(2-Amino-4-thiazolyl)-(methoxyimino)-acetyl]-amino]-4-methyl-2-oxo-1-azetidinyl]-oxy]-methyl]-phosphonsäure-methylester oder einem Salz davon.

12. Verfahren nnach Anspruch 1 zur Herstellung von [3S-[3α(Z),4β]]-[[[3-[[(2-Amino-4-thiazolyl)-(methoxyimino)-acetyl]-amino]-4-methyl-2-oxo-1-azetidinyl]-oxy]-methyl]-phosphonsäure-diäthylester oder einem Salz davon.

13. Verfahren nach Anspruch 1 zur Herstellung von [3S-[3α(Z),4β]]-[[[3-[[(2-Amino-4-thiazolyl)-(methoxyimino)-acetyl]-amino]-4-methyl-2-oxo-1-azetidinyl]-oxy]-methyl]-methylphosphinsäure oder einem Salz davon.

14. Eine Verfahren zur Herstellung einer Verbindung der Formel XI

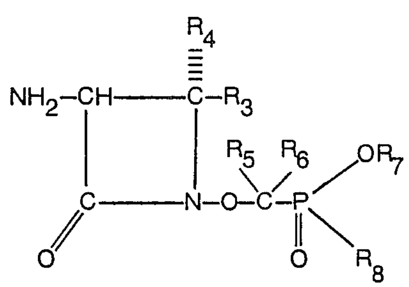

(XI)

in der

$R_3$ und $R_4$ gleich oder verschieden sind und jeweils Wasserstoff, Alkyl, Alkenyl, Alkinyl, Cycloalkyl, Phenyl, substituiertes Phenyl oder einen 4,5,6- oder 7-gliedrigen Heterocyclus bedeuten oder einer der

Reste $R_3$ und $R_4$ ein Wasserstoffatom ist und der andere Azido, Halogenmethyl, Dihalogenmethyl, Trihalogenmethyl, Alkoxycarbonyl, Alkenyl, Alkinyl, 2-Phenyläthenyl, 2-Phenyläthinyl, Carboxy oder einen der Reste

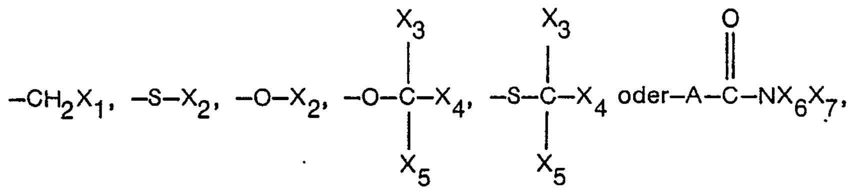

bedeutet, wobei

$X_1$ Azido, Amino, Hydroxy, Alkanoylamino, Phenylcarbonylamino, (substituiertes Phenyl)-carbonylamino, Alkylsulfonyloxy, Phenylsulfonyloxy, (substituiertes Phenyl)-sulfonyloxy, Phenyl, substituiertes Phenyl, Cyano,

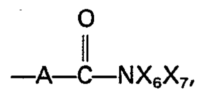

$—S—X_2$ oder $—O—X_2$ bedeutet;

$X_2$ Alkyl, substituiertes Alkyl, Phenyl, substituiertes Phenyl, Phenylalkyl, (substituiertes Phenyl)-alkyl, Alkanoyl, Phenylalkanoyl, (substituiertes Phenyl)-alkanoyl, Phenylcarbonyl, (substituiertes Phenyl)-carbonyl oder Heteroarylcarbonyl darstellt;

einer der Reste $X_3$ und $X_4$ Wasserstoff ist und der andere Wasserstoff oder Alkyl bedutet, oder $X_3$ und $X_4$ zusammen mit dem Kohlenstoffatom, an das sie gebunden sind, einen Cycloalkylrest bilden;

$X_5$ Formyl, Alkanoyl, Phenylcarbonyl, (substituiertes Phenyl)-carbonyl, Phenylalkylcarbonyl, (substituiertes Phenyl)-alkylcarbonyl, Carboxyl, Alkoxycarbonyl, Aminocarbonyl, (substituiertes Amino)-carbonyl oder Cyano darstellt;

A eine der Gruppen $—CH=CH—$, $—(CH_2)_n—$, $—CH_2—O—$, $—CH_2—NH-$ oder $—CH_2—S—CH_2—$ bedeutet;

n 0, 1 oder 2 ist; und

$X_6$ und $X_7$ gleich oder verschieden sind und jeweils Wasserstoff, Alkyl, Phenyl oder substituiertes Phenyl bedeuten, oder $X_6$ Wasserstoff ist und $X_7$ Amino, substituiertes Amino, Acylamino oder Alkoxy darstellt, oder $X_6$ und $X_7$ zusammen mit dem Stickstoffatom, an das sie gebunden sind, einen 4-, 5-, 6- oder 7-gliedrigen Heterocyclus bilden,

$R_5$ und $R_6$ gleich oder verschieden sind und jeweils Wasserstoff, Alkyl, Alkenyl, Alkinyl, Phenyl, substituiertes Phenyl, Cycloalkyl oder einen 4-, 5-, 6- oder 7-gliedrigen Heterocyclus bilden, oder $R_5$ und $R_6$ zusammen mit dem Kohlenstoffatom, an das sie gebunden sind, Cycloalkyl oder einen 4-, 5-, 6- oder 7-gliedrigen Heterocyclus bedeuten oder einer der Reste $R_5$ und $R_6$ ein Wasserstoffatom ist und der andere Azido, Halogenmethyl, Dihalogenmethyl, Trihalogenmethyl, Alkoxycarbonyl, Alkenyl, Alkinyl, 2-Phenyläthenyl, 2-Phenyläthinyl, Carboxyl, oder einen der Reste $—CH_2X_1$, $—S—X_2$, $—O—X_2$ oder

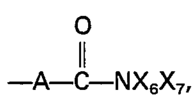

bedeutet;

$R_7$ Wasserstoff, Alkyl, substituiertes Alkyl, Phenyl, substituiertes Phenyl, 1-(Äthoxycarbonyloxy)-äthyl, 1,3-Dihydro-3-oxo-1-isobenzofuranyl, oder eine der Gruppen

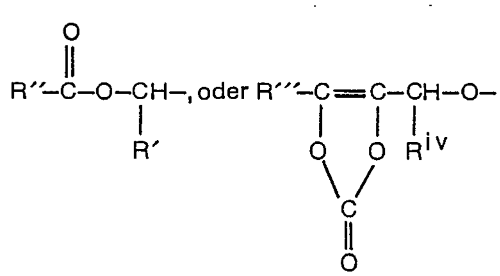

bedeutet,

wobei R′ Wasserstoff oder Alkyl ist, R″ Alkyl oder Phenyl bedeutet, R‴ Wasserstoff, Methyl oder Phenyl darstellt und $R^{iv}$ Wasserstoff ist oder zusammen mit R‴ $—(CH_2)_3—$ oder $—(CH_2)_5—$ bedeutet; und

$R_8$ Hydroxy, Alkoxy, (substituiertes Alkyl)-oxy, Phenyloxy, (substituiertes Phenyl)-oxy, Alkyl,

substituiertes Alkyl, Phenyl, substituiertes Phenyl, Heteroaryl, Amino, substituiertes Amino, Alkylthio, (substituiertes Alkyl)-thio, Phenylthio, (substituiertes Phenyl)-thio oder

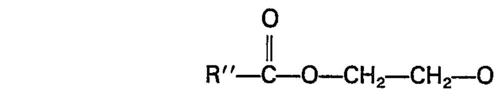

bedeutet, dadurch gekennzeichnet, daß eine Verbindung der Formel VIII

VIII

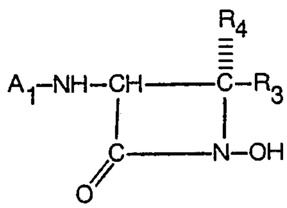

mit einer aktivierten Form einer Verbindung der Formel IX

IX

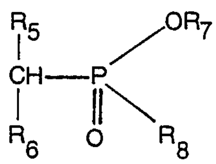

alkyliert wird und das erhaltene Produkt der Formel X

X

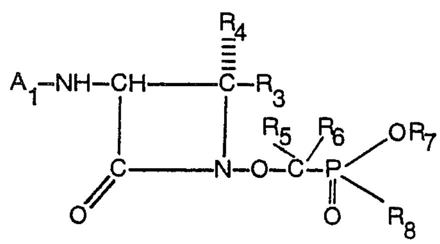

durch Entfernen der Schutzgruppen vom 3-Amino-Substituenten in eine Verbindung der Formel XI umgewandelt wird.

15. Verfahren zur Herstellung eines Arzneimittels, umfassend die Kombination einer gemäß Anspruch 1 erhaltenen Verbindung der Formel I mit einem physiologisch verträglichen Träger.

**Revendications pour les Etats contractants: BE CH DE FR GB IT LI LU NL SE**

1. Composé de formule I

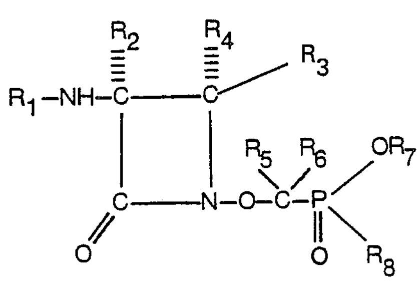

(I)

ou sel pharmaceutiquement acceptable de celui-ci, formule dans laquelle

$R_1$ est un radical acyle;

$R_2$ est l'hydrogène ou un groupement méthoxy;

$R_3$ et $R_4$ sont identiques ou différents et sont chacun l'hydrogène, un radical alkyle, alcényle, alcynyle, cycloalkyle, phényle, phényle substitué, ou un hétérocycle à 4, 5, 6 ou 7 chaînons, ou bien l'un de $R_3$ et $R_4$ est l'hydrogène et l'autre est un radical azide, halométhyle, dihalométhyle, trihalométhyle,

alcoxycarbonyle, alcényle, alcynyle, 2-phényléthényle, 2-phényléthynyle, carboxyle,

$-CH_2X_1$, $-S-X_2$, $-O-X_2$, 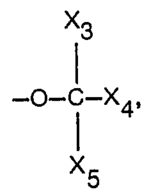, 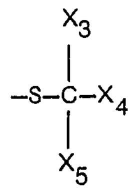 ou 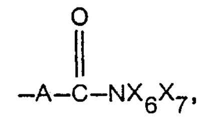,

où

$X_1$ est un groupement azide, amine, hydroxy, alcanoylamine, phénylcarbonylamine, (phényl substitué)carbonylamine, alkylsulfonyloxy, phénylsulfonyloxy, (phényl substitué) sulfonyloxy, phényle, phényle substitué, cyano,

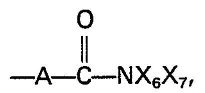,

$—S—X_2$ ou $—O—X_2$; $X_2$ est un radical alkyle, alkyle substitué, phényle, phényle substitué, phénylalkyle, (phényl substitué)alkyle, alcanoyle, phénylalcanoyle, (phényle substitué) alcanoyle, phénylcarbonyle, (phényl substitué)carbonyle, ou hétéroarylcarbonyle; l'un de $X_3$ et $X_4$ est l'hydrogène et l'autre est l'hydrogène ou un radical alkyle, ou bien $X_3$ et $X_4$, pris avec l'atome de carbone auquel ils sont fixés, forment un radical cycloalkyle; $X_5$ est un radical formyle, alcanoyle, phénylcarbonyle, (phényl substitué)carbonyle, phénylalkylcarbonyle, (phényl substitué)alkylcarbonyle, carboxyle, alcoxycarbonyle, aminocarbonyle, (amino substitué)carbonyle, ou cyano;

A est $—CH=CH—$, $—(CH_2)_n—$, $—CH_2—O—$, $—CH_2—NH—$ ou $—CH_2—S—CH_2$;

n est égal à 0, 1 ou 2; et $X_6$ et $X_7$ sont identiques ou différents et sont chacun l'hydrogène ou un radical alkyle, phényle ou phényle substitué, ou bien $X_6$ est l'hydrogène et $X_7$ est un groupement amine, amine substitué, acylamine ou alcoxy, ou encore $X_6$ et $X_7$, pris avec l'atome d'azote auquel ils sont fixés, forment un hétérocycle à 4, 5, 6 ou 7 chaînons;

$R_5$ et $R_6$ sont identiques ou différents et sont chacun l'hydrogène ou un radical alkyle, alcényle, alcynyle, phényle, phényle substitué, cycloalkyle, ou un hétérocycle à 4, 5, 6 ou 7 chaînons, ou bien $R_5$ et $R_6$, pris avec l'atome de carbone auquel ils sont fixés, forment un radical cycloalkyle ou un hétérocycle à 4, 5, 6 ou 7 chaînons, ou bien l'un de $R_5$ et $R_6$ est l'hydrogène et l'autre est un groupement azide, halométhyle, dihalométhyle, trihalométhyle, alcoxycarbonyle, alcényle, alcynyle, 2-phényléthényle, 2-phényléthynyle, carboxyle, $—CH_2X_1$, $—S—X_2$, $—O—X_2$, ou

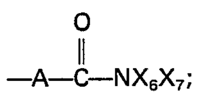;

$R_7$ est l'hydrogène ou un radical alkyle, alkyle substitué, phényle, phényle substitué, 1-(éthoxycarbonyloxy)éthyle, 1,3-dihydro-3-oxo-1-isobenzofuranyle,

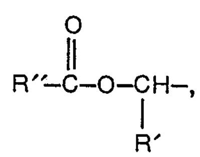, ou 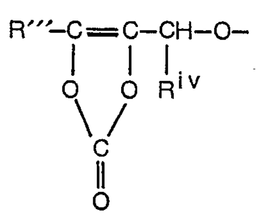

où R′ est l'hydrogène ou un radical alkyle, R″ est un radical alkyle ou phényle, R‴ est l'hydrogène ou un radical méthyle ou phényle, et $R^{iv}$ est l'hydrogène ou forme avec R‴ un groupement $—(CH_2)_3—$ ou $—(CH_2)_5$; et

$R_8$ est un groupement hydroxy, alcoxy, (alkyl substitué)oxy, phényloxy, (phényl substitué)oxy, alkyle, alkyle substitué, phényle, phényle substitué, hétéroaryle, amine, amine substitué, alkylthio, (alkyl substitué)thio, phénylthio, (phényl substitué)thio, ou

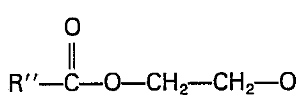

2. Composé selon la revendication 1, dans lequel $R_2$ est l'hydrogène.

3. Composé selon la revendication 1, dans lequel $R_3$ et $R_4$ sont identiques ou différents et sont chacun l'hydrogène ou un radical alkyle.

4. Composé selon la revendication 2, dans lequel $R_3$ et $R_4$ sont identiques ou différents et sont chacun l'hydrogène ou un radical alkyle.

5. Composé selon la revendication 1, dans lequel $R_5$ et $R_6$ sont identiques ou différents et sont chacun l'hydrogène ou un radical alkyle.

6. Composé selon la revendication 2, dans lequel $R_5$ et $R_6$ sont identiques ou différents et sont chacun l'hydrogène ou un radical alkyle.

7. Composé selon la revendication 1, dans lequel $R_7$ est l'hydrogène ou un radical alkyle et $R_8$ est un groupement hydroxy ou alcoxy.

8. Composé selon la revendication 2, dans lequel $R_7$ est l'hydrogène ou un radical alkyle et $R_8$ est un groupement hydroxy ou alcoxy.

9. Composé selon la revendication 1, dans lequel $R_2$ est l'hydrogène;

$R_3$ et $R_4$ sont identiques ou différents et sont chacun l'hydrogène ou un radical alkyle;

$R_5$ et $R_6$ sont identiques ou différents et sont chacun l'hydrogène ou un radical alkyle;

$R_7$ est l'hydrogène ou un radical alkyle; et

$R_8$ est un groupement hydroxy ou alcoxy.

10. Composé selon la revendication 1, qui est l'acide [3S-[3α(Z),4β]]-[[[3-[[(2-amino-4-thiazolyl) (méthoxyimino)acétyl]amino]-4-méthyl-2-oxo-1-azétidinyl]oxy]méthyl]phosphonique, ou un sel de cet acide.

11. Composé selon la revendication 1, qui est l'ester méthylique de l'acide [3S-[3α(Z),4β]]-[[[3-[[(2-amino-4-thiazolyl) (méthoxyimino)acétyl]amino]-4-méthyl-2-oxo-1-azétidinyl]oxy]méthyl]phosphonique, ou un sel de celui-ci.

12. Composé selon la revendication 1, qui est l'ester diéthylique de l'acide [3S-[3α(Z),4β]]-[[[3-[[(2-amino-4-thiazolyl) (méthoxyimino)acétyl]amino]-4-méthyl-2-oxo-1-azétidinyl]oxy]méthyl]phosphonique, ou un sel de celui-ci.

13. Composé selon la revendication 1, qui est l'acide [3S-[3α(Z),4β]]-[[[3-[[(2-amino-4-thiazolyl) (méthoxyimino)acétyl]amino]-4-méthyl-2-oxo-1-azétidinyl]oxy]méthyl]phosphonique, ou un sel de cet acide.

14. Composé de formule XI

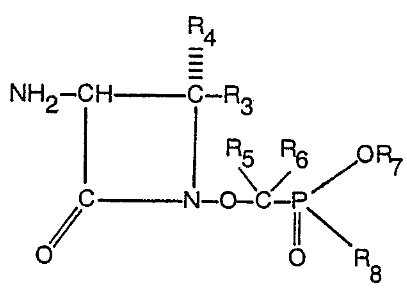

(XI)

dans laquelle

$R_3$ et $R_4$ sont identiques ou différents et sont chacun l'hydrogène, ou un radical alkyle, alcényle, alcynyle, cycloalkyle, phényle, ou phényle ou substitué, ou un hétérocycle à 4, 5, 6 ou 7 chaînons, ou bien l'un de $R_3$ et $R_4$ est l'hydrogène et l'autre est un groupement azide, halométhyle, dihalométhyle, trihalométhyle, alcoxycarbonyle, alcényle, alcynyle, 2-phényléthényle, 2-phényléthynyle, carboxyle,

$-CH_2X_1$, $-S-X_2$, $-O-X_2$, 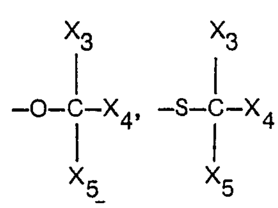 ou 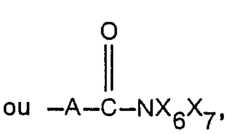,

où

$X_1$ est un groupement azide, amine, hydroxy, alcanoyl amine, phénylcarbonylamine, (phényl substitué)carbonyl amine, alkylsulfonyloxy, phénylsulfonyloxy, (phényl substitué) sulfonyloxy, phényle, phényle substitué, cyano,

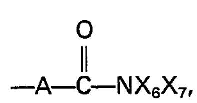,

S—$X_2$ ou —O—$X_2$; $X_2$ est un radical alkyle, alkyle substitué, phényle, phényle substitué, phénylalkyle, (phényl substitué)alkyle, alcanoyle, phénylalcanoyle, (phényle substitué) alcanoyle, phénylcarbonyle,

(phényl substitué)carbonyle, ou hétéroarylcarbonyle; l'un de $X_3$ et $X_4$ est l'hydrogène et l'autre est l'hydrogène ou un radical alkyle, ou bien $X_3$ et $X_4$, pris avec l'atome de carbone auquel ils sont fixés, forment un radical cycloalkyle; $X_5$ est un radical formyle, alcanoyle, phénylcarbonyle, (phényl substitué)carbonyle, phénylalkylcarbonyle, (phényl substitué)alkylcarbonyle, carboxyle, alcoxycarbonyle, aminocarbonyle, (amino substitué)carbonyle, ou cyano; A est $—CH=CH—$, $—(CH_2)_n—$, $—CH_2—O—$, $—CH_2—NH—$ ou $—CH_2—S—CH_2—$; n est égal à 0, 1 ou 2; et $X_6$ et $X_7$ sont identiques ou différents et sont chacun l'hydrogène ou radical alkyle, phényle ou phényle substitué, ou bien $X_6$ est l'hydrogène et $X_7$ est un groupement amine, amine substitué, acylamine ou alcoxy, ou encore $X_6$ et $X_7$ forment, avec l'atome d'azote auquel ils sont fixés, un hétérocycle à 4, 5, 6 ou 7 chaînons;

$R_5$ et $R_6$ sont identiques ou différents et sont chacun l'hydrogène ou un radical alkyle, alcényle, alcynyle, phényle, phényle substitué, ou cycloalkyle, ou un hétérocycle à 4, 5, 6 ou 7 chaînons, ou bien $R_5$ et $R_6$, forment avec l'atome de carbone auquel ils sont fixés, un radical cycloalkyle ou un hétérocycle à 4, 5, 6 ou 7 chaînons, ou bien l'un de $R_5$ et $R_6$ est l'hydrogène et l'autre est un groupement azide, halométhyle, dihalométhyle, trihalométhyle, alcoxycarbonyle, alcényle, alcynyle, 2-phényléthényle, 2-phényléthynyle, carboxyle, $—CH_2X_1$, $—S—X_2$, $—O—X_2$, ou

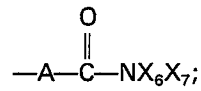

$R_7$ est l'hydrogène ou un radical alkyle, alkyle substitué, phényle, phényle substitué, 1-(éthoxycarbonyloxy)éthyle, 1,3-dihydro-3-oxo-1-isobenzofuranyle,

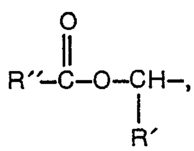 ou 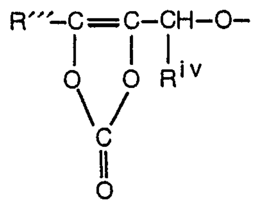

où R' est l'hydrogène ou un radical alkyle, R'' est un radical alkyle ou phényle, R''' est l'hydrogène ou un radical méthyle ou phényle, et $R^{iv}$ est l'hydrogène ou forme avec R''' un groupement $—(CH_2)_3—$ ou $—(CH_2)_5$; et

$R_8$ est un groupement hydroxy, alcoxy, (alkyl substitué)oxy, phényloxy, (phényl substitué)oxy, alkyle, alkyle substitué, phényle, phényle substitué, hétéroaryle, amine, amine substitué, alkylthio, (alkyl substitué)thio, phénylthio, (phényl substitué)thio, ou

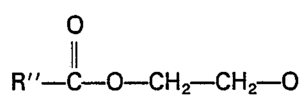

15. Composé de formule I selon la revendication 1, utilisable pour le traitement des infections bactériennes.

16. Composition pharmaceutique comprenant un composé de formule I selon la revendication 1 et un véhicule physiologiquement acceptable.

**Revendications pour l'Etat contractant: AT**

1. Procédé de préparation d'un composé de formule I

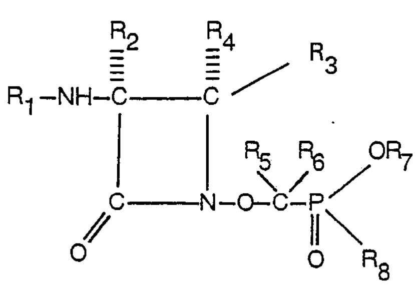

(I)

ou d'un sel pharmaceutiquement acceptable de celui-ci, formule dans laquelle

$R_1$ est un radical acyle;

$R_2$ est l'hydrogène ou un groupement méthoxy;

$R_3$ et $R_4$ sont identiques ou différents et sont chacun l'hydrogène, un radical alkyle, alcényle, alcynyle, cycloalkyle, phényle, phényle substitué, ou un hétérocycle à 4, 5, 6 ou 7 chaînons, ou bien l'un de $R_3$ et $R_4$ est l'hydrogène et l'autre est un radical azide, halométhyle, dihalométhyle, trihalométhyle, alcoxycarbonyle, alcényle, alcynyle, 2-phényléthényle, 2-phényléthynyle, carboxyle,

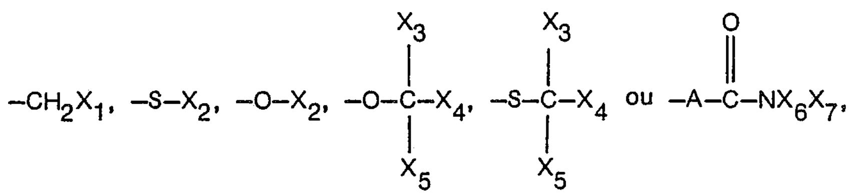

$X_1$ est un groupement azide, amine, hydroxy, alcanoylamine, phénylcarbonylamine, (phényl substitué)carbonylamine, alkylsulfonyloxy, phénylsulfonyloxy, (phényl substitué) sulfonyloxy, phényle, phényle substitué, cyano,

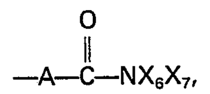

S—$X_2$ ou —O—$X_2$; $X_2$ est un radical alkyle, alkyle substitué, phényle, phényle substitué, phénylalkyle, (phényl substitué)alkyle, alcanoyle, (phényle substitué) alcanoyle, phénylcarbonyle, (phényl substitué)carbonyle, ou hétéroarylcarbonyle; l'un de $X_3$ et $X_4$ est l'hydrogène et l'autre est l'hydrogène ou un radical alkyle, ou bien $X_3$ et $X_4$, pris avec l'atome de carbone auquel ils sont fixés, forment un radical cycloalkyle; $X_5$ est un radical formyle, alcanoyle, phénylcarbonyle, (phényl substitué)carbonyule, phénylalkylcarbonyle, (phényl substitué)alkylcarbonyle, carboxyle, alcoxycarbonyle, aminocarbonyle, (amino substitué)carbonyle, ou cyano;

A est —CH=CH—, $—(CH_2)_n—$, $—CH_2—O—$, $—CH_2—NH—$ ou $—CH_2—S—CH_2$;

n est égal à 0, 1 ou 2; et $X_6$ et $X_7$ sont identiques ou différents et sont chacun l'hydrogène ou un radical alkyle, phényle ou phényle substitué, ou bien $X_6$ est l'hydrogène et $X_7$ est un groupement amine, amine substitué, acylamine ou alcoxy, ou encore $X_6$ et $X_7$, pris avec l'atome d'azote auquel ils sont fixés, forment un hétérocycle à 4, 5, 6 ou 7 chaînons;

$R_5$ et $R_6$ sont identiques ou différents et sont chacun l'hydrogène ou un radical alkyle, alcényle, alcynyle, phényle, phényle substitué, cycloalkyle, ou un hétérocycle à 4, 5, 6 ou 7 chaînons, ou bien $R_5$ et $R_6$, pris avec l'atome de carbone auquel ils sont fixés, forment un radical cycloalkyle ou un hétérocycle à 4, 5, 6 ou 7 chaînons, ou bien l'un de $R_5$ et $R_6$ est l'hydrogène et l'autre est un groupement azide, halométhyle, dihalométhyle, trihalométhyle, alcoxycarbonyle, alcényle, alcynyle, 2-phényléthényle, 2-phényléthynyle, carboxyle, $—CH_2X_1$, —S—$X_2$, —O—$X_2$, ou

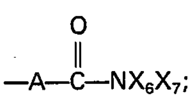

$R_7$ est l'hydrogène ou un radical alkyle, alkyle substitué, phényle, phényle substitué, 1-(éthoxycarbonyloxy)éthyle, 1,3-dihydro-3-oxo-1-isobenzofuranyle,

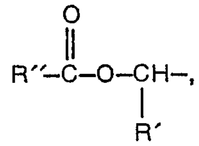 ou 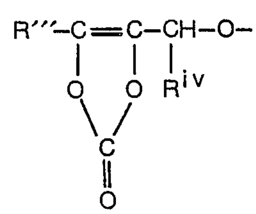

où R' est l'hydrogène ou un radical alkyle, R'' est un radical alkyle ou phényle, R''' est l'hydrogène ou un radical méthyle ou phényle, et $R^{iv}$ est l'hydrogène ou forme avec R''' un groupement $—(CH_2)_3—$ ou $—(CH_2)_5$; et

$R_8$ est un groupement hydroxy, alcoxy, (alkyl substitué)oxy, phényloxy, (phényl substitué)oxy, alkyle,

alkyle substitué, phényle, phényle substitué, hétéroaryle, amine, amine substitué, alkylthio, (alkyl substitué)thio, phénylthio, (phényl substitué)thio, ou

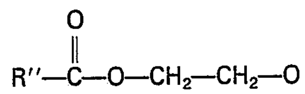

caractérisé en ce qu'on acyle un composé de formule

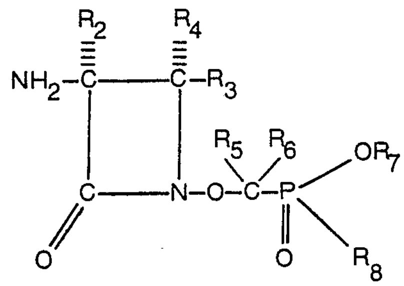

selon des techniques d'acylation connues, par exemple par réaction avec un acide carboxylique ($R_1$—OH) ou un halogénure d'acide carboxylique ou un anhydride d'acide carboxylique correspondant, et on transforme facultativement le produit obtenu en un sel pharmaceutiquement acceptable de ce produit.

2. Procédé selon la revendication 1, dans lequel $R_2$ est l'hydrogène.

3. Procédé selon la revendication 1, dans lequel $R_3$ et $R_4$ sont identiques ou différents et sont chacun l'hydrogène ou un radical alkyle.

4. Procédé selon la revendication 2, dans lequel $R_3$ et $R_4$ sont identiques ou différents et sont chacun l'hydrogène ou un radical alkyle.

5. Procédé selon la revendication 1, dans lequel $R_5$ et $R_6$ sont identiques ou différents et sont chacun l'hydrogène ou un radical alkyle.

6. Procédé selon la revendication 2, dans lequel $R_5$ et $R_6$ sont identiques ou différents et sont chacun l'hydrogène ou un radical alkyle.

7. Procédé selon la revendication 1, dans lequel $R_7$ est l'hydrogène ou un radical alkyle et $R_8$ est un groupement hydroxy ou alcoxy.

8. Procédé selon la revendication 2, dans lequel $R_7$ est l'hydrogène ou un radical alkyle et $R_8$ est un groupement hydroxy ou alcoxy.

9. Procédé selon la revendication 1, dans lequel $R_2$ est l'hydrogène;
$R_3$ et $R_4$ sont identiques ou différents et sont chacun l'hydrogène ou un radical alkyle;
$R_5$ et $R_6$ sont identiques ou différents et sont chacun l'hydrogène ou un radical alkyle;
$R_7$ est l'hydrogène ou un radical alkyle; et
$R_8$ est un groupement hydroxy ou alcoxy.

10. Procédé selon la revendication 1, pour la production de l'acide [3S-[3α(Z),4β]]-[[[3-[[(2-amino-4-thiazolyl) (méthoxyimino)acétyl]amino]-4-méthyl-2-oxo-1-azétidinyl]oxy]méthyl]phosphonique, ou d'un sel de cet acide.

11. Procédé selon la revendication 1, pour la production de l'ester méthylique de l'acide [3S-[3α(Z),4β]]-[[[3-[[(2-amino-4-thiazolyl) (méthoxyimino)acétyl]amino]-4-méthyl-2-oxo-1-azétidinyl]oxy]méthyl]phosphonique, ou d'un sel de celui-ci.

12. Procédé selon la revendication 1, pour la production de l'ester diéthylique de l'acide [3S-[3α(Z),4β]]-[[[3-[[(2-amino-4-thiazolyl) (méthoxyimino)acétyl]amino]-4-méthyl-2-oxo-1-azétidinyl]oxy]méthyl]phosphonique, ou d'un sel de celui-ci.

13. Procédé selon la revendication 1, pour la production de l'acide [3S-[3α(Z),4β]]-[[[3-[[(2-amino-4-thiazolyl) (méthoxyimino)acétyl]amino]-4-méthyl-2-oxo-azétidinyl]oxy]méthyl]phosphonique, ou d'un sel de cet acide.

14. Procédé de préparation d'un composé de formule XI

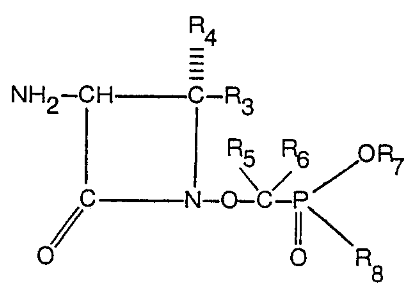

(XI)

dans laquelle

$R_3$ et $R_4$ sont identiques ou différents et sont chacun l'hydrogène, un radical alkyle, alcényle, alcynyle, cycloalkyle, phényle, ou phényle substitué, ou un hétérocycle à 4, 5, 6 ou 7 chaînons, ou bien l'un de $R_3$ et $R_4$ est l'hydrogène et l'autre est un groupement azide, halométhyle, dihalométhyle, trihalométhyle, alcoxycarbonyle, alcényle, alcynyle, 2-phényléthényle, 2-phényléthynyle, carboxyle,

$-CH_2X_1$, $-S-X_2$, $-O-X_2$, 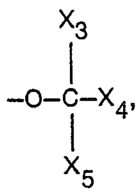, 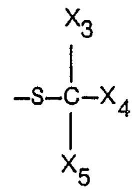 ou 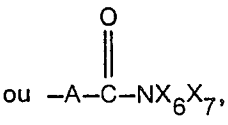,

$X_1$ est un groupement azide, amine, hydroxy, alcanoylamine, phénylcarbonylamine, (phényl substitué)carbonylamine, alkylsulfonyloxy, phénylsulfonyloxy, (phényl substitué) sulfonyloxy, phényle, phényle substitué, cyano,

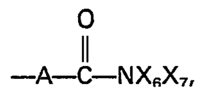,

$—S—X_2$ ou $—O—X_2$; $X_2$ est un radical alkyle, alkyle substitué, phényle, phényle substitué, phénylalkyle, (phényl substitué)alkyle, alcanoyle, phénylalcanoyle, (phényle substitué) alcanoyle, phénylcarbonyle, (phényl substitué)carbonyle, ou hétéroarylcarbonyle; l'un de $X_3$ et $X_4$ est l'hydrogène et l'autre est l'hydrogène ou un radical alkyle, ou bien $X_3$ et $X_4$, pris avec l'atome de carbone auquel ils sont fixés, forment un radical cycloalkyle; $X_5$ est un radical formyle, alcanoyle, phénylcarbonyle, (phényl substitué)carbonyule, phénylalkylcarbonyle, (phényl substitué)alkylcarbonyle, carboxyle, alcoxycarbonyle, aminocarbonyle, (amino substitué)carbonyle, ou cyano; A est $—CH=CH—$, $—(CH_2)_n—$, $—CH_2—O—$, $—CH_2—NH—$ ou $—CH_2—S—CH_2$; n est égal à 0, 1 ou 2; et $X_6$ et $X_7$ sont identiques ou différents et sont chacun l'hydrogène ou un radical alkyle, phényle ou phényle substitué, ou bien $X_6$ est l'hydrogène et $X_7$ est un groupement amine, amine substitué, acylamine ou alcoxy, ou encore $X_6$ et $X_7$, forment, avec l'atome d'azote auquel ils sont fixés, un hétérocycle à 4, 5, 6 ou 7 chaînons;

$R_5$ et $R_6$ sont identiques ou différents et sont chacun l'hydrogène, un radical alkyle, alcényle, alcynyle, phényle, phényle substitué, ou cycloalkyle, ou un hétérocycle à 4, 5, 6 ou 7 chaînons, ou bien $R_5$ et $R_6$, forment, avec l'atome de carbone auquel ils sont fixés, un radical cycloalkyle ou un hétérocycle à 4, 5, 6 ou 7 chaînons, ou bien l'un de $R_5$ et $R_6$ est l'hydrogène et l'autre est un groupement azide, halométhyle, dihalométhyle, trihalométhyle, alcoxycarbonyle, alcényle, alcynyle, 2-phényléthényle, 2-phényléthynyle, carboxyle, $—CH_2X_1$, $—S—X_2$, $—O—X_2$, ou

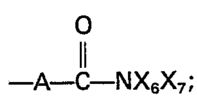;

$R_7$ est l'hydrogène ou un radical alkyle, alkyle substitué, phényle, phényle substitué, 1-(éthoxycarbonyloxy) éthyle, 1,3-dihydro-3-oxo-1-isobenzofuranyle,

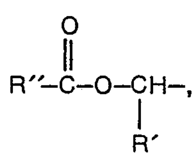, ou 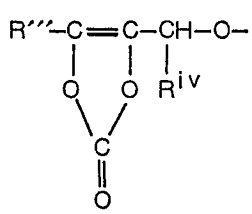

où R' est l'hydrogène ou un radical alkyle, R'' est un radical alkyle ou phényle, R''' est l'hydrogène ou un radical méthyle ou phényle, et $R^{iv}$ est l'hydrogène ou forme avec R''' un groupement $—(CH_2)_3—$ ou $—(CH_2)_5$; et

$R_8$ est un groupement hydroxy, alcoxy, (alkyl substitué)oxy, phényloxy, (phényl substitué)oxy, alkyle, alkyle substitué, phényle, phényle substitué, hétéroaryle, amine, amine substitué, alkylthio, (alkyl substitué)thio, phénylthio, (phényl substitué)thio, ou

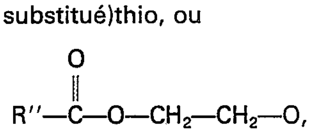,

caractérisé en ce qu'on alkyle un composé de formule VIII

VIII

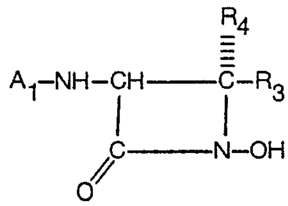

à l'aide d'une forme activée d'un composé de formule IX

IX

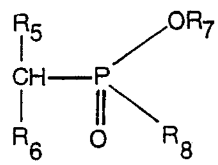

et on transforme le produit résultant, de formule X

X

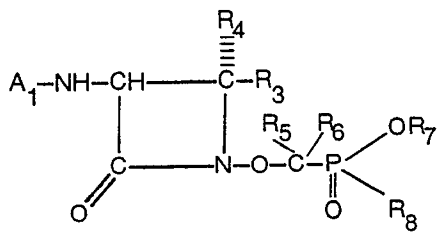

en un composé de formule XI, en supprimant la protection du substituant 3-amine.

15. Procédé de préparation d'une composition pharmaceutique consistant à associer un composé de formule I, obtenu selon la revendication 1, à un véhicule physiologiquement acceptable.